# EUROPEAN PATENT APPLICATION

(11) **EP 3 984 989 A1**
(43) Date of publication of application: **20.04.2022**
(21) Application number: 21824460.6
(22) Date of filing: 28.06.2021
(51) Int. Cl.: C07C 69/96, C07C 68/06, C07C 68/00, A61P 19/02, A61P 29/00, A61P 25/04, A61K 31/265, A61K 31/192

(54) **IBUPROFEN ESTER PRODRUG, PHARMACEUTICAL COMPOSITION AND PREPARATION METHOD AND USE**

(30) Priority: 13.08.2020 CN 202010815093
(71) Applicant: Nanjing Heron Pharmaceutical Science and Technology Co., Ltd., Nanjing, Jiangsu 211100 (CN)
(72) Inventor: YE, Hai, Nanjing Jiangsu 211100 (CN); MIN, Tao, Nanjing Jiangsu 211100 (CN); LV, Tian, Nanjing Jiangsu 211100 (CN); ZHOU, Wenliang, Nanjing Jiangsu 211100 (CN); CHEN, Xingran, Nanjing Jiangsu 211100 (CN); FENG, Yunqing, Nanjing Jiangsu 211100 (CN); MO, Meiling, Nanjing Jiangsu 211100 (CN); WANG, Jialin, Nanjing Jiangsu 211100 (CN)
(74) Representative: HGF
(86) International application number: PCT/CN2021/102752
(87) International publication number: WO 2022/033202

(57) **Abstract**

Provided is an ibuprofen ester prodrug represented by Structural Formula (1), a racemate, stereoisomer or pharmaceutically acceptable salt or solvate thereof, or a solvate of a pharmaceutically acceptable salt thereof. Further provided are a method for preparing the compound, a pharmaceutical composition containing the compound, and an application of the compound in preparation of nonsteroidal anti-inflammatory drugs. The pharmaceutical composition containing the ibuprofen ester prodrug may be prepared into fat emulsion injection preparations. The ibuprofen ester prodrug has good stability and good pharmacokinetic properties and overcomes the problems of ibuprofen such as a short half-life, poor stability, irritation, and incompatibility.

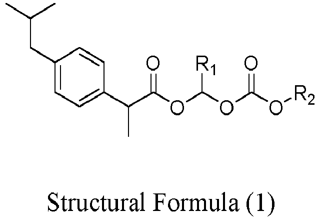

## Description

### CROSS-REFERENCE TO RELATED APPLICATION(S)

This application claims priority to Chinese Patent Application No. 202010815093.9 filed with China National Intellectual Property Administration (CNIPA) on Aug. 13, 2020 and titled *IBUPROFEN ESTER PRODRUG, PHARMACEUTICAL COMPOSITION, PREPARATION METHOD AND APPLICATION,* the disclosure of which is incorporated herein by reference in its entirety.

### TECHNICAL FIELD

The present application belongs to the field of drugs and, in particular, relates to an ibuprofen ester prodrug, a preparation method and application thereof, and a pharmaceutical composition containing the ibuprofen ester prodrug.

### BACKGROUND

Ibuprofen whose chemical name is 2-(4-isobutylphenyl)propionic acid has analgesic, anti-inflammatory, anti-rheumatic, antipyretic and other effects. Ibuprofen is currently the most widely used nonsteroidal anti-inflammatory drug (NSAID) around the world. Ibuprofen has been clinically used as an anti-inflammatory and analgesic drug for nearly 30 years and is considered to be the safest and most effective one among many NSAIDs. Ibuprofen is an over-the-counter drug and a recommended first step drug of an analgesic ladder . Ibuprofen functions mainly by preventing cyclooxygenase from competing for a hydrogen atom of arachidonic acid so as to prevent the peroxidation of arachidonic acid and ultimately prevent the production of prostaglandins and blood coagulation factors.

Ibuprofen has a short half-life and needs to be frequently administered to maintain a therapeutic concentration, causing some adverse reactions. For example, oral tablets of ibuprofen have the problems of a short half-life and low bioavailability in the gastrointestinal tract. Therefore, the oral tablets of ibuprofen need to be frequently administered for a long time, which increases side effects on the gastrointestinal tract and causes certain damages to the kidney. This limits the clinical application of ibuprofen.

Ibuprofen contains a chiral carbon in structure. Commercially available ibuprofen is racemic ibuprofen which contains 50% levorotatory ibuprofen and 50% dextrorotatory ibuprofen (that is, S-ibuprofen). Studies show that levorotatory ibuprofen has weak anti-inflammatory, antipyretic and analgesic effects, dextrorotatory ibuprofen is 28 times stronger than levorotatory ibuprofen, and levorotatory ibuprofen may cause various adverse reactions such as gastrointestinal toxicity, water and sodium retention, decreased renal perfusion and allergic reactions.

Studies show that S-ibuprofen has significantly better clinical effects (Adam et al., J. Pharm. Pharakol, 28, 257 and Jamali et al., Pharmac Res. 1988, 5, 44). Compared with a racemic mixture, an S-isomer can quickly reach a therapeutic concentration in the blood. Though racemic ibuprofen has been accepted as an anti-inflammatory and analgesic drug by a majority of patients, racemic ibuprofen, if frequently administered at a high dosage as an effective drug for the treatment of rheumatism and rheumatoid arthritis and other chronic diseases for a long time, will increase the side effects on the gastrointestinal tract and even cause gastrointestinal bleeding and cause certain damages to the kidney, which greatly limits its clinical application. Therefore, single chiral S-ibuprofen is worth studying.

Currently, commercially available dexibuprofen includes only tablets, capsules, injections and suppositories. Oral preparations have the problems of uneven absorption, a strong first-pass effect, and low bioavailability. Dexibuprofen has a short half-life and needs to be frequently administered to maintain the therapeutic concentration, has poor patient compliance, and causes some adverse reactions. Currently, commercially available injections are administered intravenously and irritate injection sites intensely. Therefore, it is necessary to develop a novel dexibuprofen drug with improved and reduced toxic side effects, which is the inevitably demand for expanding the clinical application of the drug.

Shanbhag et al. have described that the gastrointestinal irritation from NSAIDs containing carboxyl groups such as ibuprofen is mainly caused by the carboxyl groups on molecules. A prodrug prepared from ibuprofen can improve patient compliance [J Pharmaceutical Sciences, 1992, 81(2): 149-154]. The prodrug of ibuprofen mainly focuses on the amidation or esterification of the carboxyl group. Since a human body is rich in biological enzymes that hydrolyze esterases, to esterify the carboxyl group on ibuprofen is an effective method for preparing the prodrug of ibuprofen.

### SUMMARY

The present application provides an ibuprofen ester prodrug, a pharmaceutical composition, a preparation method and an application.

In the present application, the carboxyl group of racemic ibuprofen or S-ibuprofen is derivatized so that a series of ester prodrugs are designed and synthesized, and also prepared into fat emulsion injection preparations, which overcomes some problems in the clinical application of the existing ibuprofen and can achieve the purpose of prolonging a half-life, improving stability, improving efficacy, reducing toxic side effects, or the like.

In a first aspect, the present application provides an ibuprofen ester prodrug, a racemate, stereoisomer or pharmaceutically acceptable salt or solvate thereof, or a solvate of a pharmaceutically acceptable salt thereof, where the ibuprofen ester prodrug is a compound represented by Structural Formula (1):

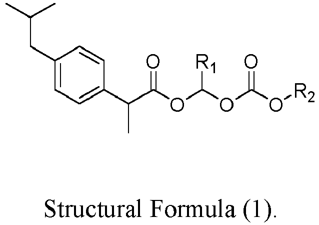

In Structural Formula (1), R₁ and R₂ are identical or different and are each independently selected from C₁₋₁₀ alkyl, C₂₋₁₀ alkenyl, C₂₋₁₀ alkynyl, C₃₋₈ cycloalkyl, 5-10 membered heterocyclic groups, C₆₋₁₀ aryl, and 5-10 membered heteroaryl groups; wherein C₁₋₁₀ alkyl, C₂₋₁₀ alkenyl, C₂₋₁₀ alkynyl, C₁₋₁₀ alkoxy, C₃₋₈ cycloalkyl, C₃₋₈ cycloalkyloxy, 5-10 membered heterocyclic groups, C₆₋₁₀ aryl, and 5-10 membered heteroaryl groups can be optionally substituted by one, two or more halogens, hydroxyl groups, amino groups, C₁₋₈ alkyl groups, C₂₋₈ alkenyl groups, C₂₋₈ alkynyl groups, C₁₋₈ alkoxy groups, C₆₋₁₀ aryl groups optionally substituted by C₁₋₁₀ alkyl, or 5-10 membered heteroaryl groups optionally substituted by C₁₋₁₀ alkyl.

According to an embodiment of the present application, in Structural Formula (1), R₁ and R₂ are identical or different and are each independently selected from C₁₋₆ alkyl, C₂₋₆ alkenyl, C₂₋₆ alkynyl, C₃₋₈ cycloalkyl, 5-8 membered heterocyclic groups, C₆₋₈ aryl, and 5-8 membered heteroaryl groups; where C₁₋₆ alkyl, C₂₋₆ alkenyl, C₂₋₆ alkynyl, C₁₋₆ alkoxy, C₃₋₈ cycloalkyl, C₃₋₈ cycloalkyloxy, 5-8 membered heterocyclic groups, C₆₋₈ aryl, and 5-8 membered heteroaryl groups can be optionally substituted by one, two or more halogens, hydroxyl groups, amino groups, C₁₋₆ alkyl groups, C₂₋₆ alkenyl groups, C₂₋₆ alkynyl groups, C₁₋₆ alkoxy groups, C₆₋₈ aryl groups optionally substituted by C₁₋₆ alkyl, or 5-8 membered heteroaryl groups optionally substituted by C₁₋₆ alkyl.

According to an embodiment of the present application, in Structural Formula (1), R₁ and R₂ are identical or different and are each independently selected from C₁₋₆ alkyl, C₂₋₆ alkenyl, C₂₋₆ alkynyl, and C₃₋₈ cycloalkyl, where C₁₋₆ alkyl, C₂₋₆ alkenyl, C₂₋₆ alkynyl, and C₃₋₈ cycloalkyl can be optionally substituted by one, two or more fluorine, chlorine, bromine, iodine, hydroxyl groups, amino groups, or phenyl groups optionally substituted by C₁₋₆ alkyl.

According to an embodiment of the present application, in Structural Formula (1), R₁ is methyl, ethyl, propyl, isopropyl, n-butyl, isobutyl, tert-butyl, cyclopropyl, cyclobutyl, cyclopentyl, or cyclohexyl; and R₂ is methyl, ethyl, propyl, isopropyl, n-butyl, tert-butyl, isobutyl, cyclopropyl, cyclobutyl, cyclopentyl, or cyclohexyl.

According to an embodiment of the present application, the compound represented by Structural Formula (1) is selected from the following compounds:

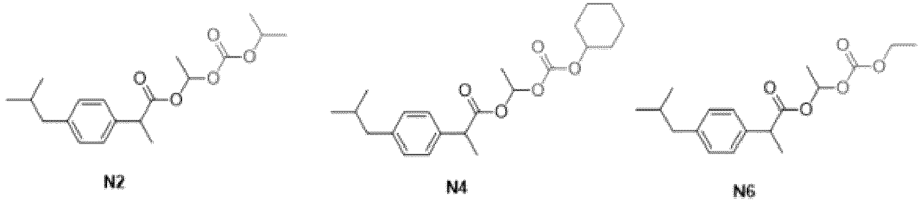

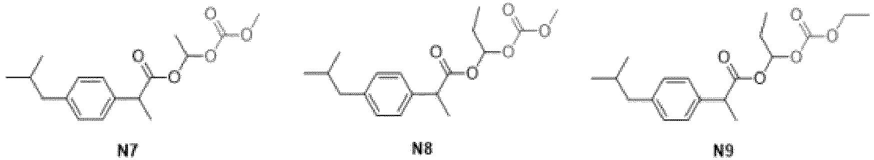

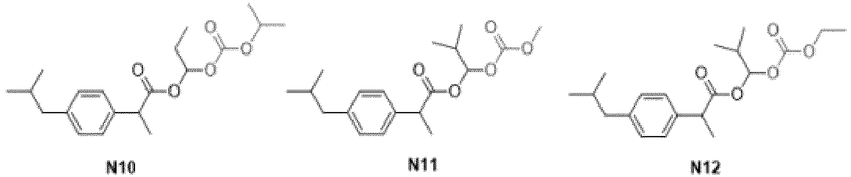

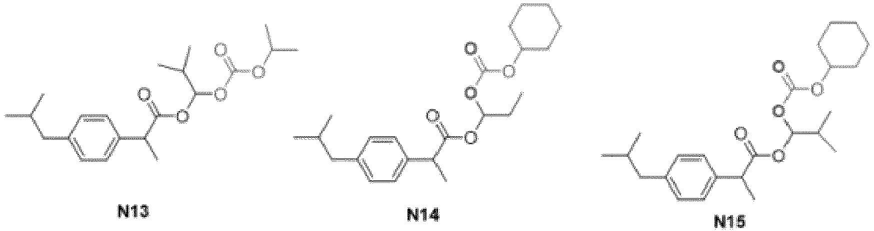

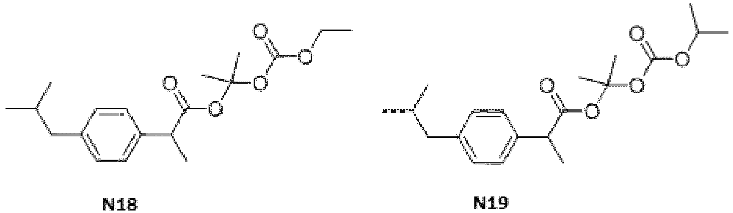

According to an embodiment of the present application, the compound represented by Structural Formula (I) is further preferred selected from Compounds N2, N6, N9, N10, N18, N19 and dextrorotatory enantiomers thereof, that is, isomers whose carbon at position 1 is in an S configuration. Such compounds have by-products with low toxicity after metabolism in the human body as well as a faster metabolic rate, which are more suitable for development into injection preparations. These compounds may be administered through subcutaneous injection, intramuscular injection, intravenous injection, intravenous infusion, or the like. The dextrorotatory enantiomers of Compounds N2, N6, N9, N10, N18 and N19 are shown below:

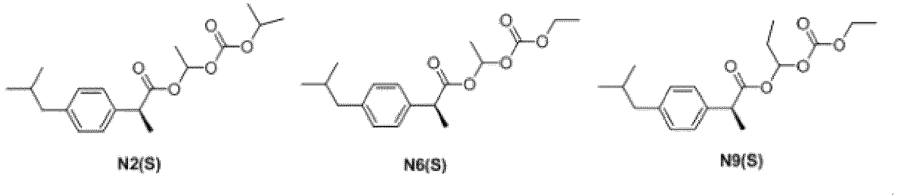

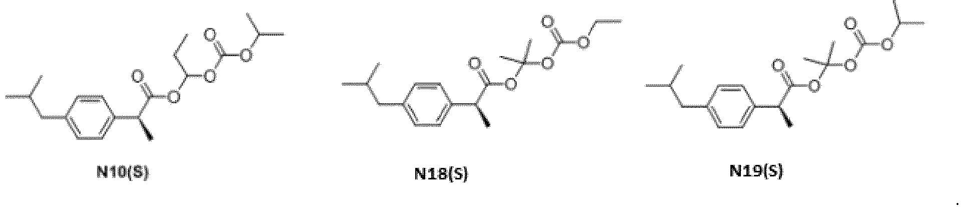

According to an embodiment of the present application, the compound represented by Structural Formula (I) is further selected from Compounds N4, N14 and dextrorotatory enantiomers thereof, that is, isomers whose carbon at position 1 is in an S configuration. Such compounds have by-products with low toxicity after metabolism in the human body as well as a slow metabolic rate, which are more suitable for development into long-acting sustained-release preparations. The dextrorotatory enantiomers of Compounds N4 and N14 are shown below:

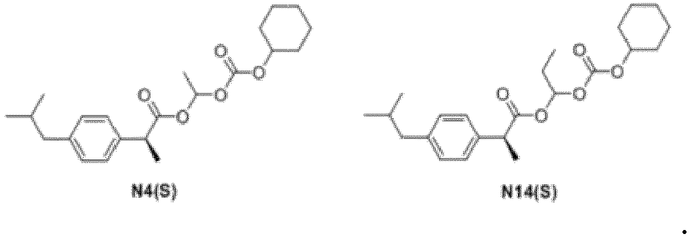

In a second aspect, the present application provides a method for preparing a compound represented by Structural Formula (1), a racemate, stereoisomer or pharmaceutically acceptable salt or solvate thereof, or a solvate of a pharmaceutically acceptable salt thereof. The method includes a step of reacting Compound 1 with an organic carbonate represented by Structural Formula (2):

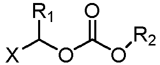

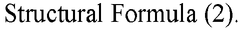

In Structural Formula (2), X is chlorine, bromine, or iodine, and R₁ and R₂ have meanings as defined above.

According to an embodiment of the present application, Compound 1 is racemic ibuprofen or ibuprofen with an S or R configuration, that is, (±)-2-(4-isobutylphenyl)propionic acid, (S)-2-(4-isobutylphenyl)propionic acid, or (R)-2-(4-isobutylphenyl)propionic acid.

According to an embodiment of the present application, the method for preparing the compound represented by Structural Formula (1) includes a step of reacting Compound 1 with a compound represented by Structural Formula (2), where the reaction formula is shown as follows: 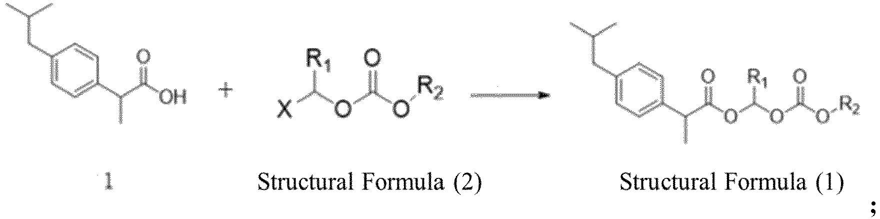 wherein, X, R₁ and R₂ have meanings as defined above.

According to an embodiment of the present application, the method for preparing the compound represented by Structural Formula (1) is conducted in the presence of an acid-binding agent.

According to an embodiment of the present application, the method for preparing the compound represented by Structural Formula (1) including:
putting the ibuprofen and the acid-binding agent in a reaction vessel, adding a reaction solvent to mix, then adding a compound represented by Structural Formula (2) 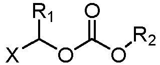 (wherein, X, R₁ and R₂ have meanings as defined above) to the reaction vessel, and stirring the reaction solution after the addition. Then, extracting the reaction solution and washing, drying an organic phase and concentrating, and purifying through column chromatography to obtain a target compound.

According to an embodiment of the present application, NaI/KI or a phase transfer catalyst is added or heating is performed, so as to accelerate the reaction process. For example, when X in the compound represented by Structural Formula (2) is chlorine, the reaction process may be accelerated in such manner.

According to an embodiment of the present application, the reaction may be conducted at a temperature of -5-80 °C for a time of 0.5-24 h; the acid-binding agent used may be an inorganic base such as NaOH, KOH, K₂CO₃, KHCO₃, Na₂CO₃ and NaHCO₃ or an organic base such as one, two or more of triethylamine, pyridine, DMAP, DIEA or DBU; and the reaction solvent may be one, two or more of acetone, dichloromethane, trichloromethane, carbon tetrachloride, tetrahydrofuran, acetonitrile, DMF, DMAc or ether. If the phase transfer catalyst is used, tetrabutylammonium bromide, 18-crown-6 ether or the like may be used.

In a third aspect, the present application further provides a method for preparing a halogenated organic carbonate (X being chlorine or iodine) represented by Structural Formula (2). The method including: firstly reacting an organic aldehyde R₁CHO (wherein R₁ has the meaning as defined above) with triphosgene at a low temperature to obtain a chloroalkyl formate intermediate, and then reacting the chloroalkyl formate with an organic alcohol R₂-OH (where R₂ has the meaning as defined above) to obtain a chlorinated organic carbonate; and optionally, further reacting the chlorinated organic carbonate with NaI to obtain an iodo organic carbonate; where the reaction formula is: 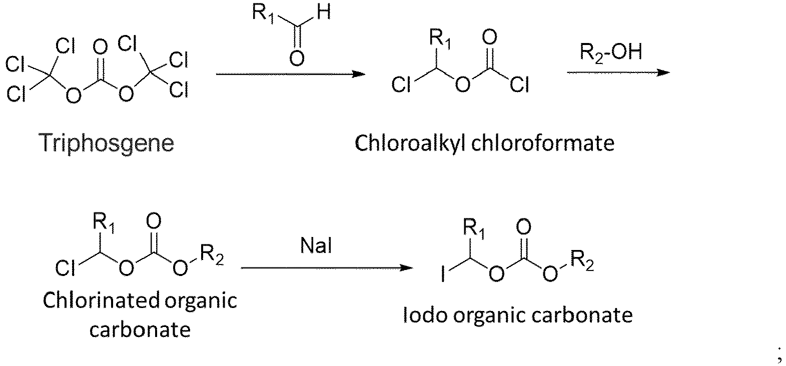 wherein, R₁ and R₂ each have meanings as defined above.

According to an embodiment of the present application, the method for preparing the chlorinated organic carbonate (X being Cl or I) represented by Structural Formula (2) includes the following steps:
step 1: triphosgene, an acid-binding agent and a reaction solvent are added to a reaction vessel placed in a low temperature environment and N₂ protection, an aldehyde compound 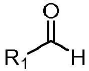 (wherein R₁ has the meaning as defined above) is slowly added dropwise to the reaction vessel, the reaction is continued for a certain period of time at 0 °C after the addition, and pumped under reduced pressure (the removed gas is absorbed by lye), and the residual solution is concentrated under reduced pressure at room temperature for the solvent to be removed and then distilled so that a chloroalkyl chloroformate is obtained;
step 2: the chloroalkyl chloroformate obtained in the preceding step and an alkyl alcohol R₂-OH (wherein R₂ has the meaning as defined above) are added to a water-free and oxygen-free reaction vessel containing the reaction solvent, placed in an ice bath of 0 °C, and slowly added with the acid-binding agent, and the reaction solution is warmed to room temperature and stirred to continue the reaction after the addition,; then, the reaction solution is washed, dried, and concentrated under reduced pressure so that the chlorinated organic carbonate (X being Cl) represented by Structural Formula (2) is obtained; and
step 3: under N₂ protection, the chlorinated organic carbonate prepared in the preceding step, anhydrous NaI, and a phase transfer catalyst or desiccant are placed in a reaction flask, added with the solvent, and mixed, the reaction is heated, and the reaction solution is cooled to room temperature, washed with 5%-25% sodium thiosulfate, water, and saturated salt solution, dried, and concentrated for the solvent to be removed or further distilled so that the iodo organic carbonate (X being I) of Structural Formula (2) is obtained.

According to an embodiment of the present application, the reaction solvent in the preceding step 1, step 2 and step 3 may be one, two or more of acetone, dichloromethane, trichloromethane, carbon tetrachloride, ether, acetonitrile, ethyl acetate, DMF, toluene, tetrahydrofuran, or DMAc; the acid-binding agent used may be pyridine, triethylamine, DIEA, DBU or NaOH, KOH, K₂CO₃, KHCO₃, Na₂CO₃, NaHCO₃, etc.; the reaction may be conducted for a time of 0.5-12 h. The low temperature environment in step 1 is -50 °C to 0 °C, for example, -40 °C to 0 °C, -30 °C to 0 °C. Exemplarily, the low temperature environment is -20 °C or -2 °C.

According to an embodiment of the present application, the phase transfer catalyst used in the preceding step 3 may be 18-crown-6, tetrabutylammonium bromide, or the like; the desiccant that may be added to the reaction may be CaCl₂, MgSO₄, Na₂SO₄, or the like; the reaction solvent may be acetonitrile, ethyl acetate, DMF, toluene, tetrahydrofuran, DMAc, or the like; the reaction may be conducted at a temperature of 25-100 °C for a time of 1-12 h.

In a fourth aspect, the present application provides an application of a compound represented by Structural Formula (1), a racemate, stereoisomer or pharmaceutically acceptable salt or solvate thereof, or a solvate of a pharmaceutically acceptable salt thereof in preparation of a drug.

According to an embodiment of the present application, the drug may be used for treatment of one or more of the following diseases: rheumatoid arthritis, low back pain, migraine, neuralgia, periarthritis of shoulder or osteoarthritis, anti-inflammation and/or analgesia of neck-shoulder-wrist syndromes, analgesia and/or anti-inflammation after surgery, trauma or tooth extraction, and antipyretic and/or analgesia of acute upper respiratory tract inflammation.

According to an embodiment of the present application, the drug is a nonsteroidal anti-inflammatory drug.

In a fifth aspect, the present application provides a pharmaceutical composition containing a compound represented by the above Structural Formula (1), a racemate, stereoisomer or pharmaceutically acceptable salt or solvate thereof, or a solvate of a pharmaceutically acceptable salt thereof.

According to an embodiment of the present application, the pharmaceutical composition further includes a pharmaceutically acceptable excipient. The pharmaceutical composition may be an oral preparation such as tablets, capsules and granules and may also be injections, eye drops, gels, creams, ointments, cataplasm, or the like.

The pharmaceutical composition of the present application may be administered through injection. Injection manners include intravenous injection, intramuscular injection, subcutaneous injection, intradermal injection, acupoint injection, and the like. The pharmaceutical composition of the present application may be prepared into preparations suitable for injection, such as an injection, an injectable emulsion, and a powder injection. The injectable emulsion may be a fat emulsion injection.

In a sixth aspect, the present application further provides a fat emulsion injection containing a compound represented by Structural Formula (1), a racemate, stereoisomer or pharmaceutically acceptable salt or solvate thereof, or a solvate of a pharmaceutically acceptable salt thereof, an oily substance, and an emulsifier.

The oily substance suitable for use in the present application is an oil ester for injection and selected from soybean oil, safflower oil, cottonseed oil, olive oil, sesame oil, coconut oil, castor oil, sea buckthorn oil, evening primrose oil, corn oil, oleum fructus bruceae, perilla oil, grape seed oil, tea oil, palm oil, peanut oil, medium chain oil (medium chain triglyceride oil), long chain triglyceride oil, ethyl oleate, acetylated monoglyceride, propylene glycol diester, glyceryl linoleate or glyceryl polyethylene glycol laurate or a combination of two or more selected therefrom.

According to an embodiment of the present application, the emulsifier is selected from a natural emulsifier such as one of soybean lecithin, egg-yolk lecithin, hydrogenated lecithin, or saturated and unsaturated C₁₂₋₁₈ fatty acyl phosphatidylcholine or a combination of two or more selected therefrom, for example, egg-yolk lecithin and/or soybean phospholipid, or a synthetic non-ionic emulsifier such as Tween-80, poloxamer-188, or a combination thereof.

According to an embodiment of the present application, the fat emulsion injection may be further added with other pharmaceutically acceptable excipients such as a co-emulsifier, an isosmotic adjusting agent, a pH adjusting agent, an antioxidant, a stabilizer, and a metal chelating agent.

The co-emulsifier is oleic acid, sodium oleate, cholesterol, cholic acid, sodium cholate, sodium deoxycholate, sodium glycocholate, or egg-yolk phosphatidylglycerol (EPG) or a combination of two or more selected therefrom.

The isosmotic adjusting agent is selected from glycerin, sorbitol, glucose, maltose, mannitol, or propylene glycol or a combination of two or more selected therefrom.

The pH adjusting agent is selected from sodium hydroxide, hydrochloric acid, phosphoric acid, a phosphate, citric acid, a citrate, citric acid, a citrate, acetic acid, an acetate, glycine, or lysine or a combination of two or more selected therefrom. According to an embodiment of the present application, the pH adjusting agent adjusts the pH between 5.5 and 8.5, preferably 6.0 and 8.0.

The antioxidant is selected from sodium sulfite, sodium bisulfite, or sodium metabisulfite or a combination of two or more selected therefrom.

The stabilizer is one of oleic acid, phosphatidylglycerol, or sodium oleate or a combination of two or more selected therefrom.

The metal chelating agent is ethylenediaminetetraacetic acid (EDTA) or a sodium salt of ethylenediaminetetraacetic acid (EDTA-Na), especially ethylenediaminetetraacetic acid disodium salt.

According to an embodiment of the present application, the fat emulsion injection may be a fat emulsion injection or a freeze-dried emulsion. The fat emulsion injection further contains water for injection. The fat emulsion injection is freeze-dried so that the freeze-dried emulsion is obtained. Before being freeze-dried, the fat emulsion injection is added with a freeze-drying protective agent, where the freeze-drying protective agent may be one of lactose, sucrose, mannitol, dextran 20, dextran 40, dextran 70, xylitol, sorbitol, or trehalose or a combination of two, three or more selected therefrom.

The fat emulsion injection prepared in the present application is suitable to be administered through parenteral administration which includes intravenous, intraarterial, subcutaneous, intraperitoneal, or intramuscular injection or infusion or through intracranial administration such as intrathecal or intracerebroventricular administration. The fat emulsion injection may be administered parenterally in the form of a large bolus or may be administered through, for example, a continuous infusion pump. Alternatively, the fat emulsion injection may be administered through intracranial administration such as intrathecal or intracerebroventricular administration. Commonly used containers of injections include glass ampoules, vials, plastic ampoules, prefilled syringes, or the like.

The fat emulsion injection in the present application contains 0.01-400 mg of active ingredient per milliliter; preferably, it contains 5-300 mg of active ingredient per milliliter; most preferably, it is a unit mass preparation containing 10-200 mg of active ingredient per milliliter.

The fat emulsion injection in the present application has an average particle size within a range of 10-1000 nm, for example, a range of 20-800 nm, a range of 30-500 nm, a range of 40-400 nm, a range of 50-300 nm, or the like.

In a seventh aspect, the present application further provides a method for preparing the fat emulsion injection. The method includes the following steps: a compound represented by Structural Formula (1), a racemate, stereoisomer or pharmaceutically acceptable salt or solvate thereof, or a solvate of a pharmaceutically acceptable salt thereof is mixed uniformly with an oil phase substance so that an oil phase is obtained; an optional co-emulsifier, isosmotic adjusting agent, antioxidant, stabilizer, metal chelating agent, and freeze-drying protective agent are dispersed into an appropriate amount of water for injection so that a water phase is obtained; the oil phase, the water phase, and an emulsifier are mixed and emulsified so that an initial emulsion is obtained; then, the initial emulsion is added with water for injection to volume, further emulsified, and added with a pH adjusting agent to adjust the pH so that the fat emulsion injection is obtained.

According to an embodiment of the present application, the method for preparing the fat emulsion injection optionally further includes a step of freeze-drying the fat emulsion injection to obtain a freeze-dried emulsion.

### Beneficial Effects

(1) In the present application, the carboxyl group of racemic ibuprofen is derivatized so that a series of ibuprofen ester prodrugs are designed and prepared, which overcomes the problems of ibuprofen such as a short half-life, poor stability, irritation, and incompatibility. The compound of the present application exhibits good pharmacokinetic properties through in vitro plasma tests. Moreover, the compound itself has relatively high physical and chemical stability. For example, the purity of the compound remains basically unchanged in high-temperature tests (storage at 60 °C for 5-10 days).
(2) The main pharmacological activity of ibuprofen comes from dexibuprofen. In the present application, a dexibuprofen ester prodrug with a (1S) configuration is synthesized with dexibuprofen as a raw material. Studies found that the dexibuprofen ester prodrug has a faster hydrolysis rate in plasma than a levorotatory ibuprofen prodrug and can produce more dexibuprofen. It is known in the art that levorotatory ibuprofen has weak anti-inflammatory, antipyretic and analgesic effects, dextrorotatory ibuprofen (in an S configuration) is 28 times stronger than levorotatory ibuprofen (in an R configuration), and levorotatory ibuprofen may cause various adverse reactions such as gastrointestinal toxicity, water and sodium retention, decreased renal perfusion and allergic reactions. Therefore, it is of great significance to study and prepare the ibuprofen ester prodrug in the S configuration, that is, the dextrorotatory enantiomer of the compound represented by Structural Formula (1) in the present application.
(3) It is found through further tests that after the dextrorotatory enantiomer of the ibuprofen ester prodrug of the present application is placed in a water phase or an oil phase for 10 days at a high temperature of 80 °C, the related substance and an isomer can both maintain relatively high stability.
(4) A series of ibuprofen ester prodrugs prepared in the present application contain a carbonate structure on a side chain, which improves fat solubility. The compound having this structure is an oily substance and can be dissolved in an oily substance to be prepared into the fat emulsion injection. Injection manners include intravenous injection, intramuscular injection, subcutaneous injection, intradermal injection, acupoint injection, and the like. The damages of a nonsteroidal anti-inflammatory drug such as traditional ibuprofen to gastrointestinal mucosae are avoided, thereby increasing the bioavailability of the drug.

### BRIEF DESCRIPTION OF DRAWINGS

FIG. 1 shows a degradation rate of an S-isomer of a compound of the present application in human plasma.
FIG. 2 shows a level of ibuprofen produced through metabolism of an S-isomer of a compound of the present application in human plasma.
FIG. 3 shows a liquid chromatogram of degradation of Compound N6(S) of the present application in human plasma.
FIG. 4 shows a liquid chromatogram of degradation of Compound N9(S) of the present application in human plasma.
FIG. 5 shows a liquid chromatogram of degradation of Compound N2(S) of the present application in human plasma.

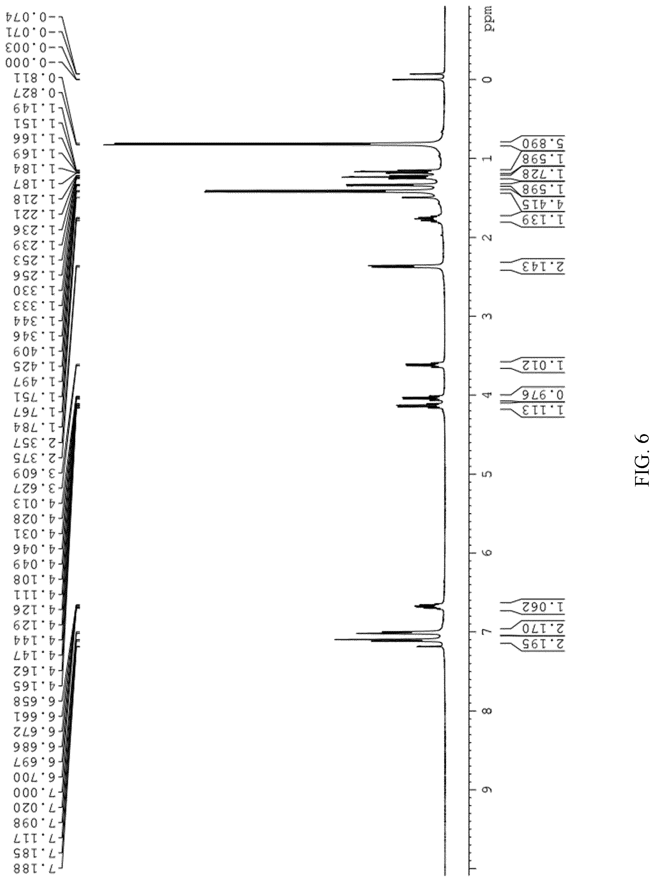

FIG. 6 shows a ¹H NMR spectrum of Compound N6(S) of the present application.

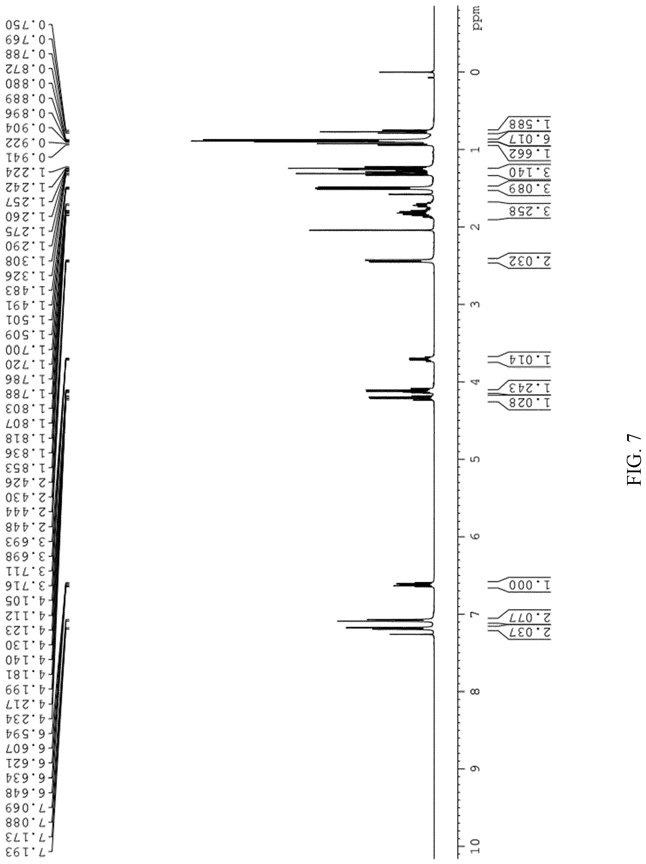

FIG. 7 shows a ¹H NMR spectrum of Compound N9(S) of the present application.
FIG. 8 shows plasma concentration-time curves of injections of dexibuprofen and a dexibuprofen ester derivative N9(S).
FIG. 9 shows plasma concentration-time curves of injections of dexibuprofen, a dexibuprofen ester derivative N9(S), and side chain chiral compounds N9(S)A and N9(S)B.

### DETAILED DESCRIPTION

Technical solutions of the present application are described below in more detail in conjunction with specific examples. It is to be understood that the following examples are merely intended to exemplarily describe and explain the present application and not to be construed as limiting the scope of the present application. Any techniques achieved based on the preceding content of the present application are within the scope of the present application.

Unless otherwise specified, raw materials and reagents used in the following examples are all commercially available products or can be prepared by known methods.

### General formula for synthesis:

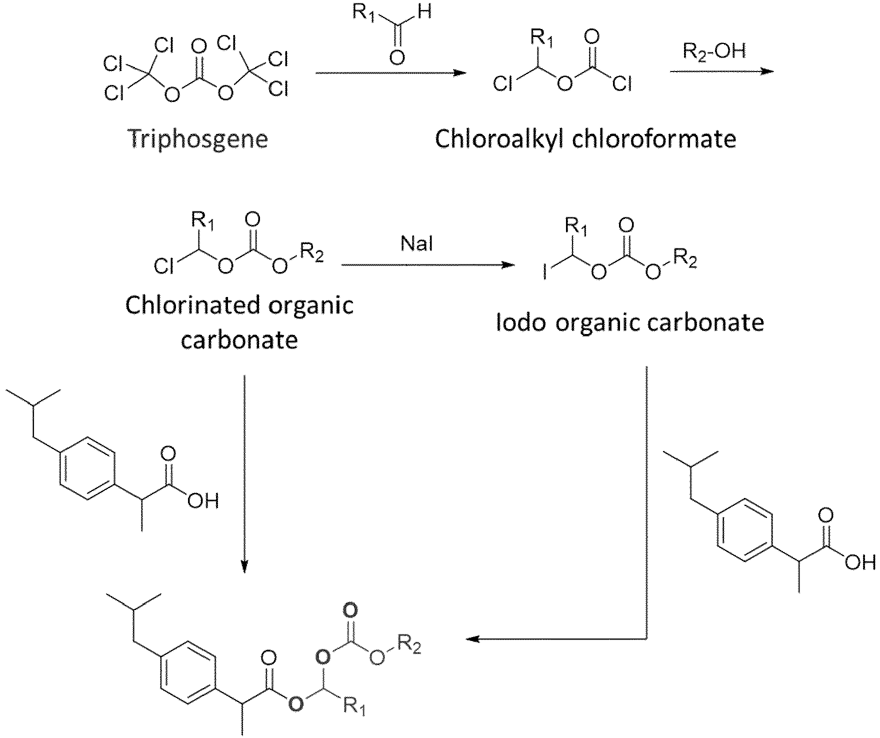

wherein, X, R₁ and R₂ each have meanings as defined above.

### General method for synthesis:

**Synthesis of a chloroalkyl chloroformate:** triphosgene, an acid-binding agent and a reaction solvent are added to a reaction vessel placed in a low temperature environment and N₂ protection, an aldehyde compound 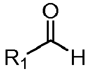 is slowly added dropwise to the reaction vessel, the reaction is continued for a certain period of time at 0 °C after the addition, and pumped under reduced pressure (the removed gas is absorbed by lye), and the residual solution is concentrated under reduced pressure at room temperature for the solvent to be removed and then distilled so that the chloroalkyl chloroformate is obtained.

**Synthesis of a chlorinated organic carbonate:** the chloroalkyl chloroformate obtained in the preceding step and an alkyl alcohol R₂-OH are separately added to a water-free and oxygen-free reaction vessel containing the reaction solvent, placed in an ice bath of 0 °C, and slowly added with the acid-binding agent (such as pyridine), and then the reaction is transferred to room temperature and stirred for a certain period of time after the addition. Then, the reaction solution is washed, dried, and concentrated under reduced pressure so that the chlorinated organic carbonate (X being Cl) is obtained.

**Synthesis of an iodo organic carbonate:** under N₂ protection, the chlorinated organic carbonate, anhydrous NaI, and a phase transfer catalyst or desiccant are placed in a reaction flask, added with the solvent, and mixed, and the reaction is heated for a certain period of time and then cooled to room temperature, washed with 5%-25% sodium thiosulfate, water, and saturated salt solution, dried, and concentrated for the solvent to be removed or further distilled so that the iodo organic carbonate is obtained.

Abbreviations used in the context have the following meanings: Ar is short for argon, DCM is short for dichloromethane, Py is short for pyridine, TATB is short for triaminotrinitrobenzene, EA is short for ethyl acetate, DBU is short for 1,8-diazabicycloundec-7-ene, and PE is short for petroleum ether.

### Example 1: Synthesis of Compound N2

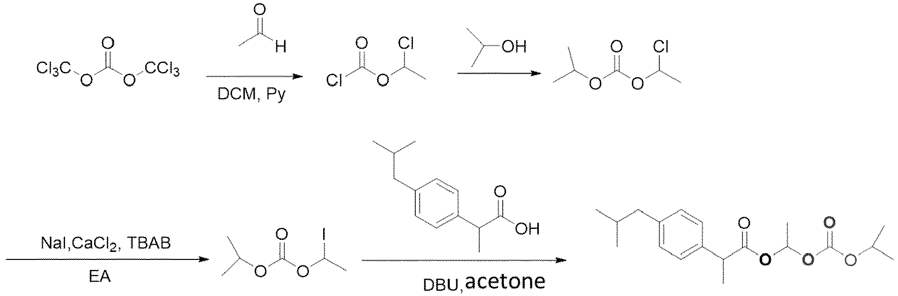

**Synthesis of 1-chloroethyl chloroformate:** triphosgene (10 g, 33.70 mmol) was weighed into a 100 mL three-necked reaction flask and added with 15 mL of anhydrous dichloromethane, and the reaction flask was purged with Ar three times, transferred to a cold trap of -20 °C, and stirred continuously. Py (0.54 g, 6.83 mmol) was diluted with 5 mL of dichloromethane and then added to the reaction flask. Paracetaldehyde (3.49 g, 26.41 mmol) was weighed and slowly added dropwise to the reaction flask, and after the drop, the temperature of the cold trap was set to -2 °C and the reaction was continued for 20 h. The reaction system was subjected to gas extraction for 5 min with a water pump that was connected with a flask containing an aqueous solution of KOH, and then transferred out of the cold trap. The solution was concentrated under reduced pressure for DCM to be removed and then distilled to obtain 2.29 g of a colorless oil with a yield of 55%.

**Synthesis of 1-chloroethyl isopropyl carbonate:** 1-chloroethyl chloroformate (0.776 g, 8.26 mmol) was weighed into a dry two-necked reaction flask, added with 10 mL of anhydrous DCM, and stirred continuously. Isopropanol (0.744 g, 12.39 mmol) was weighed into the above reaction flask. The reaction flask was transferred to an ice-water bath and stirred continuously. Pyridine (0.784 g, 9.91 mmol) was weighed and slowly added dropwise to the above reaction flask with a white solid appearing, and after the drop, the reaction flask was transferred to room temperature for a reaction of 1 h. 10 mL of water was added to the reaction flask, layers were separated, and the DCM layer was washed with 5% KHSO₄ to a pH of 3-4, washed with water to nearly neutral, washed with saturated salt solution, and dried over anhydrous sodium sulfate. The resulting product was concentrated under reduced pressure to obtain 0.811 g of a colorless oil, which was directly used in the next step without purification.

**Synthesis of 1-iodoethyl isopropyl carbonate:** 1-chloroethyl isopropyl carbonate (0.811 g, 4.88 mmol), CaCl₂ (0.184 g, 1.66 mmol), TBAB (0.031 g, 0.10 mmol), and NaI (0.614 g, 4.10 mmol) were weighed into a 100 mL two-necked reaction flask, added with 50 mL of ethyl acetate, and heated to reflux for 3 h. 20 mL of water was added to the reaction flask, layers were separated, and the EA layer was washed twice with 5% sodium thiosulfate, washed with saturated salt solution, dried over anhydrous sodium sulfate, and concentrated to obtain a yellow oil, which was directly used in the next step without purification.

**Synthesis of N2:** ibuprofen (0.677 g, 3.28 mmol) was weighed into a dry single-necked reaction flask, added with 10 mL of acetone, and stirred to be dissolved, 1-iodoethyl isopropyl carbonate (1.269 g, 4.92 mmol) was added, and DBU (0.449 g, 2.95 mmol) was slowly added. The reaction was conducted for 1 h at 40 °C and continued for 2-6 h with the temperature maintained. EA and water were added to the reaction, layers were separated, and the EA layer was washed with 5% NaHCO₃, water, and saturated salt solution, dried, and concentrated. The resulting product was subjected to flash column chromatography (PE/EA= 60:1) to obtain a colorless oil (0.666 g, 1.98 mmol) with a yield of 60.4%.
¹H NMR (400 MHz, CDCl₃) δ 7.19-7.06 (m, 4H), 6.79-6.72 (m, 1H), 4.91-4.75 (m, 1H), 3.72-3.66 (m, 1H), 2.45-2.42(m, 2H), 1.89-1.79 (m, 1H), 1.50-1.47 (m, 5H), 1.41-1.27 (m,3H), 1.26-1.20 (m, 4H), 0.889 (d, *J=* 6.6 Hz, 6H);
ESI-MS m/z =359.1, [M+Na]⁺.

### Example 2: Synthesis of Compound N2(S)

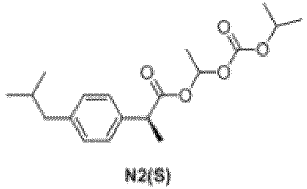

The operations were the same as those in Example 1 except that ibuprofen was replaced with S-ibuprofen.
¹H NMR (400 MHz, CDCl₃) δ 7.19-7.06 (m, 4H), 6.79-6.72 (m, 1H), 4.91-4.75 (m, 1H), 3.72-3.66 (m, 1H), 2.45-2.42 (m, 2H), 1.89-1.79 (m, 1H), 1.50-1.47 (m, 5H), 1.41-1.27 (m,3H), 1.26-1.20 (m, 4H), 0.889 (d, *J=* 6.6 Hz, 6H);
ESI-MS m/z =359.1, [M+Na]⁺.

### Example 3: Synthesis of Compound N4

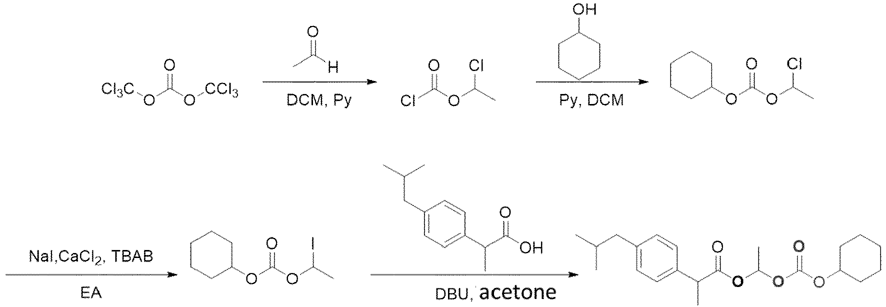

**Synthesis of 1-chloroethyl chloroformate:** triphosgene (10 g, 33.70 mmol) was weighed into a 100 mL three-necked reaction flask and added with 15 mL of anhydrous dichloromethane, and the reaction flask was purged with Ar three times, transferred to a cold trap of -20 °C, and stirred continuously. Py (0.54 g, 6.83 mmol) was diluted with 5 mL of dichloromethane and then added to the reaction flask. Paracetaldehyde (3.49 g, 26.41 mmol) was weighed and slowly added dropwise to the reaction flask, and after the drop, the temperature of the cold trap was set to -2 °C and the reaction was continued for 20 h. The reaction system was subjected to gas extraction for 5 min with a water pump that was connected with a flask containing an aqueous solution of KOH, and then transferred out of the cold trap. The solution was concentrated under reduced pressure for DCM to be removed and then distilled to obtain 2.29 g of a colorless oil with a yield of 55%.

**Synthesis of 1-chloroethyl cyclohexyl carbonate:** 1-chloroethyl chloroformate (0.544 g, 2.47 mmol) was weighed into a dry two-necked reaction flask, added with 10 mL of anhydrous DCM, and stirred continuously. Cyclohexanol (0.381 g, 3.80 mmol) was weighed into the above reaction flask. The reaction flask was transferred to an ice-water bath and stirred continuously. Pyridine (0.219 g, 2.77 mmol) was weighed and slowly added dropwise to the above reaction flask with a white solid appearing, and after the drop, the reaction flask was transferred to room temperature for a reaction of 1 h. 10 mL of water was added to the reaction flask, layers were separated, and the DCM layer was washed with 5% KHSO₄ to a pH of 3-4, washed with water to nearly neutral, washed with saturated salt solution, and dried over anhydrous sodium sulfate. The resulting product was concentrated under reduced pressure to obtain 0.680 g of a colorless oil, which was directly used in the next step without purification.

**Synthesis of 1-iodoethyl cyclohexyl carbonate:** 1-chloroethyl cyclohexyl carbonate (0.680 g, 2.18 mmol), CaCl₂ (0.082 g, 0.74 mmol), TBAB (0.016 g, 0.05 mmol), and NaI (0.274 g, 1.83 mmol) were weighed into a 100 mL two-necked reaction flask, added with 50 mL of ethyl acetate, and heated to reflux for 3 h. 20 mL of water was added to the reaction flask, layers were separated, and the EA layer was washed twice with 5% sodium thiosulfate, washed with saturated salt solution, dried over anhydrous sodium sulfate, and concentrated to obtain a yellow oil, which was directly used in the next step without purification.

**Synthesis of N4:** ibuprofen (0.342 g, 1.66 mmol) was weighed into a dry single-necked reaction flask, added with 10 mL of acetone, and stirred to be dissolved, 1-iodoethyl cyclohexyl carbonate (0.815 g, 2.09 mmol) was added, and DBU (0.228 g, 1.50 mmol) was slowly added. The reaction was conducted for 1 h at 40 °C and continued for 2-6 h with the temperature maintained. EA and water were added to the reaction, layers were separated, and the EA layer was washed with 5% NaHCO₃, water, and saturated salt solution, dried, and concentrated. The resulting product was subjected to flash column chromatography (PE/EA = 60:1) to obtain a colorless oil (0.520 g, 1.65 mmol) with a yield of 80.3%.
¹H NMR (400 MHz, CDCl₃) δ 7.19-7.06 (m, 4H), 6.79-6.72 (m, 1H), 4.65-4.49 (m, 1H), 3.71-3.66 (m, 1H), 2.45-2.42 (m, 2H), 1.87-1.70 (m, 5H), 1.49-1.48 (m, 6H), 1.41-1.30 (m, 6H), 0.90-0.88 (m, 6H);
ESI-MS m/z =399.2, [M+Na]⁺.

### Example 4: Synthesis of Compound N6

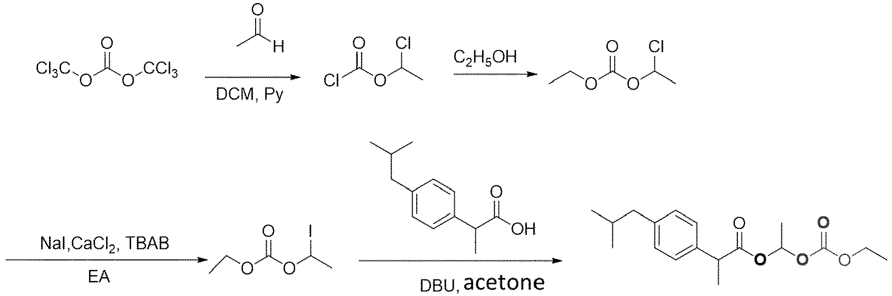

**Synthesis of 1-chloroethyl chloroformate:** triphosgene (10 g, 33.70 mmol) was weighed into a 100 mL three-necked reaction flask and added with 15 mL of anhydrous dichloromethane, and the reaction flask was purged with Ar three times, transferred to a cold trap of -20 °C, and stirred continuously. Py (0.54 g, 6.83 mmol) was diluted with 5 mL of dichloromethane and then added to the reaction flask. Paracetaldehyde (3.49 g, 26.41 mmol) was weighed and slowly added dropwise to the reaction flask, and after the drop, the temperature of the cold trap was set to -2 °C and the reaction was continued for 20 h. The reaction system was subjected to gas extraction for 5 min with a water pump that was connected with a flask containing an aqueous solution of KOH, and then transferred out of the cold trap. The solution was concentrated under reduced pressure for DCM to be removed and then distilled to obtain 2.29 g of a colorless oil with a yield of 55%.

**Synthesis of 1-chloroethyl ethyl carbonate:** 1-chloroethyl chloroformate (1.026 g, 10.92 mmol) was weighed into a dry two-necked reaction flask, added with 10 mL of anhydrous DCM, and stirred continuously. Ethanol (0.754 g, 16.38 mmol) was weighed into the above reaction flask. The reaction flask was transferred to an ice-water bath and stirred continuously. Pyridine (1.037 g, 13.11 mmol) was weighed and slowly added dropwise to the above reaction flask with a white solid appearing, and after the drop, the reaction flask was transferred to room temperature for a reaction of 1 h. 10 mL of water was added to the reaction flask, layers were separated, and the DCM layer was washed with 5% KHSO₄ to a pH of 3-4, washed with water to nearly neutral, washed with saturated salt solution, and dried over anhydrous sodium sulfate. The resulting product was concentrated under reduced pressure to obtain 1.104 g of a colorless oil, which was directly used in the next step without purification.

**Synthesis of 1-iodoethyl ethyl carbonate:** 1-chloroethyl ethyl carbonate (1.104 g, 7.24 mmol), CaCl₂ (0.274 g, 2.47 mmol), TBAB (0.047 g, 0.15 mmol), and NaI (0.914 g, 6.10 mmol) were weighed into a 100 mL two-necked reaction flask, added with 50 mL of ethyl acetate, and heated to reflux for 3 h. 20 mL of water was added to the reaction flask, layers were separated, and the EA layer was washed twice with 5% sodium thiosulfate, washed with saturated salt solution, dried over anhydrous sodium sulfate, and concentrated to obtain a yellow oil, which was directly used in the next step without purification.

**Synthesis of N6:** ibuprofen (0.625 g, 3.03 mmol) was weighed into a dry single-necked reaction flask, added with 10 mL of acetone, and stirred to be dissolved, 1-iodoethyl ethyl carbonate (1.110 g, 4.55 mmol) was added, and DBU (0.415 g, 2.73 mmol) was slowly added. The reaction was conducted for 1 h at 40 °C and continued for 2-6 h with the temperature maintained. EA and water were added to the reaction, layers were separated, and the EA layer was washed with 5% NaHCO₃, water, and saturated salt solution, dried, and concentrated. The resulting product was subjected to flash column chromatography (PE/EA= 60:1) to obtain a colorless oil (0.573 g, 1.78 mmol) with a yield of 58.7%.
¹H NMR (400 MHz, CDCl₃) δ 7.19-7.07 (m, 4H), 6.78-6.72 (m, 1H), 4.24-4.08 (m, 2H), 3.72-3.66 (m, 1H), 2.44 (d, 2H, *J=* 7.1 Hz), 1.89-1.79 (m, 1H), 1.43-1.30 (m, 6H), 1.26-1.14 (m, 3H), 0.82 (d, 6H, *J* = 6.6 Hz);
ESI-MS m/z =345.1, [M+Na]⁺.

### Example 5: Synthesis of Compound N6(S)

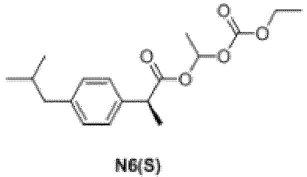

The operations were the same as those in Example 4 except that ibuprofen was replaced with S-ibuprofen.
¹H NMR (400 MHz, CDCl₃) δ 7.19-7.07 (m, 4H), 6.78-6.72 (m, 1H), 4.24-4.08 (m, 2H), 3.72-3.66 (m, 1H), 2.44 (d, 2H, *J=* 7.1 Hz), 1.89-1.79 (m, 1H), 1.43-1.30 (m, 6H), 1.26-1.14 (m, 3H), 0.82 (d, 6H, *J* = 6.6 Hz);
ESI-MS m/z =345.1, [M+Na]⁺.

### Example 6: Synthesis of Compound N7

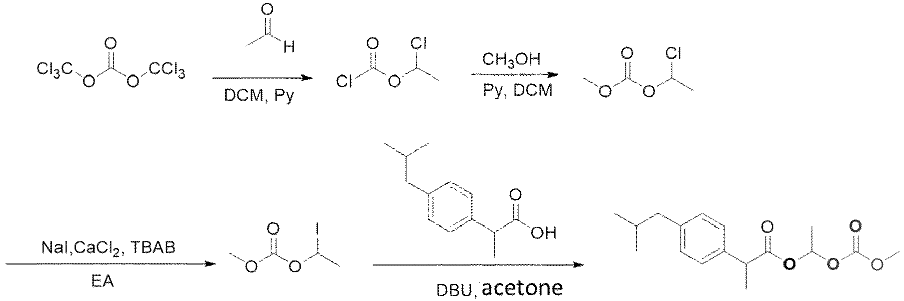

**Synthesis of 1-chloroethyl chloroformate:** triphosgene (10 g, 33.70 mmol) was weighed into a 100 mL three-necked reaction flask and added with 15 mL of anhydrous dichloromethane, and the reaction flask was purged with Ar three times, transferred to a cold trap of -20 °C, and stirred continuously. Py (0.54 g, 6.83 mmol) was diluted with 5 mL of dichloromethane and then added to the reaction flask. Paracetaldehyde (3.49 g, 26.41 mmol) was weighed and slowly added dropwise to the reaction flask, and after the drop, the temperature of the cold trap was set to -2 °C and the reaction was continued for 20 h. The reaction system was subjected to gas extraction for 5 min with a water pump that was connected with a flask containing an aqueous solution of KOH, and then transferred out of the cold trap. The solution was concentrated under reduced pressure for DCM to be removed and then distilled to obtain 2.29 g of a colorless oil with a yield of 55%.

**Synthesis of 1-chloroethyl methyl carbonate:** 1-chloroethyl chloroformate (0.660 g, 7.04 mmol) was weighed into a dry two-necked reaction flask, added with 10 mL of anhydrous DCM, and stirred continuously. Methanol (0.338 g, 10.56 mmol) was weighed into the above reaction flask. The reaction flask was transferred to an ice-water bath and stirred continuously. Pyridine (0.685 g, 8.66 mmol) was weighed and slowly added dropwise to the above reaction flask with a white solid appearing, and after the drop, the reaction flask was transferred to room temperature for a reaction of 1 h. 10 mL of water was added to the reaction flask, layers were separated, and the DCM layer was washed with 5% KHSO₄ to a pH of 3-4, washed with water to nearly neutral, washed with saturated salt solution, and dried over anhydrous sodium sulfate. The resulting product was concentrated under reduced pressure to obtain 0.779 g of a colorless oil, which was directly used in the next step without purification.

**Synthesis of 1-iodoethyl methyl carbonate:** 1-chloroethyl methyl carbonate (0.779 g, 5.64 mmol), CaCl₂ (0.213 g, 1.92 mmol), TBAB (0.035 g, 0.11 mmol), and NaI (0.700 g, 4.67 mmol) were weighed into a 100 mL two-necked reaction flask, added with 50 mL of ethyl acetate, and heated to reflux for 3 h. 20 mL of water was added to the reaction flask, layers were separated, and the EA layer was washed twice with 5% sodium thiosulfate, washed with saturated salt solution, dried over anhydrous sodium sulfate, and concentrated to obtain a yellow oil, which was directly used in the next step without purification.

**Synthesis of N7:** ibuprofen (0.590 g, 2.86 mmol) was weighed into a dry single-necked reaction flask, added with 10 mL of acetone, and stirred to be dissolved, 1-iodoethyl methyl carbonate (0.986 g, 4.29 mmol) was added, and DBU (0.391 g, 2.57 mmol) was slowly added. The reaction was conducted for 1 h at 40 °C and continued for 2-6 h with the temperature maintained. EA and water were added to the reaction, layers were separated, and the EA layer was washed with 5% NaHCO₃, water, and saturated salt solution, dried, and concentrated. The resulting product was subjected to flash column chromatography (PE/EA= 60:1) to obtain a colorless oil (0.508 g, 1.65 mmol) with a yield of 57.7%.
¹H NMR (400 MHz, CDCl₃) δ 7.22-7.08 (m, 4H), 6.91-6.74 (m, 1H), 4.26-4.11 (d, *J=* 28.8 Hz, 3H), 3.75-3.70 (m, 1H), 2.43 (d, 2H, *J=* 7.1 Hz), 1.90-1.77 (m, 1H), 1.52-1.38 (m, 6H), 0.91 (d, 6H, *J =* 6.6 Hz);
ESI-MS m/z =309.2, [M+H]⁺.

### Example 7: Synthesis of Compound N8

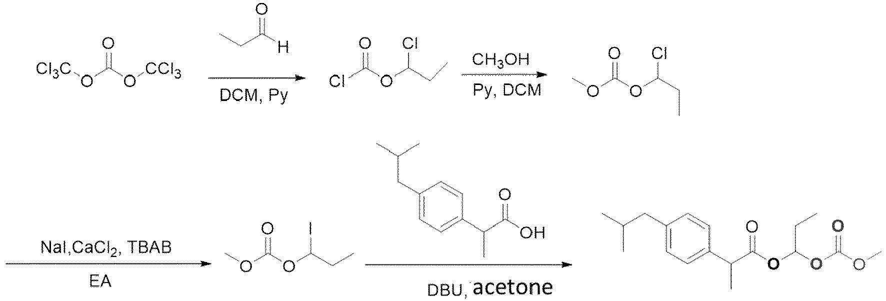

**Synthesis of 1-chloropropyl chloroformate:** triphosgene (10 g, 33.70 mmol) was weighed into a 100 mL three-necked reaction flask and added with 15 mL of anhydrous dichloromethane, and the reaction flask was purged with Ar three times, transferred to a cold trap of -20 °C, and stirred continuously. Py (0.54 g, 6.83 mmol) was diluted with 5 mL of dichloromethane and then added to the reaction flask. N-propionaldehyde (4.09 g, 70.42 mmol) was weighed and slowly added dropwise to the reaction flask, and after the drop, the temperature of the cold trap was set to -2 °C and the reaction was continued for 20 h. The reaction system was subjected to gas extraction for 5 min with a water pump that was connected with a flask containing an aqueous solution of KOH, and then transferred out of the cold trap. The solution was concentrated under reduced pressure for DCM to be removed and then distilled to obtain 6.44 g of a colorless oil with a yield of 58.6%.

**Synthesis of 1-chloropropyl methyl carbonate:** 1-chloropropyl chloroformate (1.0 g, 6.41 mmol) was weighed into a dry two-necked reaction flask, added with 10 mL of anhydrous DCM, and stirred continuously. Methanol (0.308 g, 9.62 mmol) was weighed into the above reaction flask. The reaction flask was transferred to an ice-water bath and stirred continuously. Pyridine (0.623 g, 7.88 mmol) was weighed and slowly added dropwise to the above reaction flask with a white solid appearing, and after the drop, the reaction flask was transferred to room temperature for a reaction of 1 h. 10 mL of water was added to the reaction flask, layers were separated, and the DCM layer was washed with 5% KHSO₄ to a pH of 3-4, washed with water to nearly neutral, washed with saturated salt solution, and dried over anhydrous sodium sulfate. The resulting product was concentrated under reduced pressure to obtain 0.784 g of a colorless oil, which was directly used in the next step without purification.

**Synthesis of 1-iodopropyl methyl carbonate:** 1-chloropropyl methyl carbonate (0.784 g, 5.16 mmol), CaCl₂ (0.184 g, 1.66 mmol), TBAB (0.031 g, 0.10 mmol), and NaI (0.649 g, 4.33 mmol) were weighed into a 100 mL two-necked reaction flask, added with 50 mL of ethyl acetate, and heated to reflux for 3 h. 20 mL of water was added to the reaction flask, layers were separated, and the EA layer was washed twice with 5% sodium thiosulfate, washed with saturated salt solution, dried over anhydrous sodium sulfate, and concentrated to obtain a yellow oil, which was directly used in the next step without purification.

**Synthesis of N8:** ibuprofen (0.660 g, 3.20 mmol) was weighed into a dry single-necked reaction flask, added with 10 mL of acetone, and stirred to be dissolved, 1-iodopropyl methyl carbonate (0.742 g, 4.88 mmol) was added, and DBU (0.434 g, 2.85 mmol) was slowly added dropwise under the condition of an ice bath. The reaction was conducted for 3 h at 40 °C. DCM and water were added to the reaction, layers were separated, and the DCM layer was washed with 5% NaHCO₃, water, and saturated salt solution, dried, and concentrated. The resulting product was subjected to flash column chromatography (PE/EA = 60:1) to obtain 0.775 g of a colorless oil with a yield of 75.3%.
¹H NMR (400 MHz, CDCl₃) δ 7.19-7.07 (m, 4H), 6.65-6.59 (m, 1H), 4.24-4.09 (d, *J=* 32.5 Hz, 3H), 3.73-3.68 (m, 1H), 2.45-2.43 (m, 2H), 1.89-1.68 (m, 3H), 1.51-1.48 (m, 3H), 0.94-0.75 (m, 9H);
ESI-MS m/z =345.3, [M+Na]⁺.

### Example 8: Synthesis of Compound N9

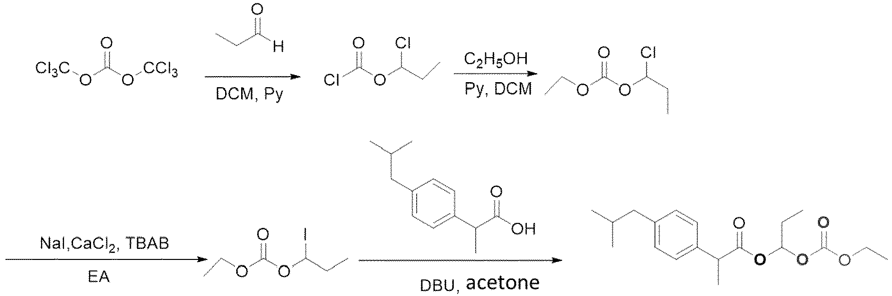

**Synthesis of 1-chloropropyl chloroformate:** triphosgene (10 g, 33.70 mmol) was weighed into a 100 mL three-necked reaction flask and added with 15 mL of anhydrous dichloromethane, and the reaction flask was purged with Ar three times, transferred to a cold trap of -20 °C, and stirred continuously. Py (0.54 g, 6.83 mmol) was diluted with 5 mL of dichloromethane and then added to the reaction flask. N-propionaldehyde (4.09 g, 70.42 mmol) was weighed and slowly added dropwise to the reaction flask, and after the drop, the temperature of the cold trap was set to -2 °C and the reaction was continued for 20 h. The reaction system was subjected to gas extraction for 5 min with a water pump that was connected with a flask containing an aqueous solution of KOH, and then transferred out of the cold trap. The solution was concentrated under reduced pressure for DCM to be removed and then distilled to obtain 6.44 g of a colorless oil with a yield of 58.6%.

**Synthesis of 1-chloropropyl ethyl carbonate:** 1-chloropropyl chloroformate (1.0 g, 6.41 mmol) was weighed into a dry two-necked reaction flask, added with 10 mL of anhydrous DCM, and stirred continuously. Ethanol (0.443 g, 9.62 mmol) was weighed into the above reaction flask. The reaction flask was transferred to an ice-water bath and stirred continuously. Pyridine (0.623 g, 7.88 mmol) was weighed and slowly added dropwise to the above reaction flask with a white solid appearing, and after the drop, the reaction flask was transferred to room temperature for a reaction of 1 h. 10 mL of water was added to the reaction flask, layers were separated, and the DCM layer was washed with 5% KHSO₄ to a pH of 3-4, washed with water to nearly neutral, washed with saturated salt solution, and dried over anhydrous sodium sulfate. The resulting product was concentrated under reduced pressure to obtain 0.766 g of a colorless oil, which was directly used in the next step without purification.

**Synthesis of 1-iodopropyl ethyl carbonate:** 1-chloropropyl ethyl carbonate (0.766 g, 4.61 mmol), CaCl₂ (0.174 g, 1.57 mmol), TBAB (0.027 g, 0.09 mmol), and NaI (0.580 g, 3.87 mmol) were weighed into a 100 mL two-necked reaction flask, added with 50 mL of ethyl acetate, and heated to reflux for 3 h. 20 mL of water was added to the reaction flask, layers were separated, and the EA layer was washed twice with 5% sodium thiosulfate, washed with saturated salt solution, dried over anhydrous sodium sulfate, and concentrated to obtain a yellow oil, which was directly used in the next step without purification.

**Synthesis of N9:** ibuprofen (0.411 g, 1.99 mmol) was weighed into a dry single-necked reaction flask, added with 10 mL of acetone, and stirred to be dissolved, 1-iodopropyl ethyl carbonate (0.777 g, 3.01 mmol) was added, and DBU (0.267 g, 1.75 mmol) was slowly added dropwise under an ice bath. The reaction was conducted for 3 h at 40 °C. DCM and water were added to the reaction, layers were separated, and the DCM layer was washed with 5% NaHCO₃, water, and saturated salt solution, dried, and concentrated. The resulting product was subjected to flash column chromatography (PE/EA= 60:1) to obtain 0.49 g of a colorless oil with a yield of 73.2%.
¹H NMR (400 MHz, CDCl₃) δ 7.19-7.07 (m, 4H), 6.65-6.59 (m, 1H), 4.24-4.09 (m, 2H), 3.73-3.68 (m, 1H), 2.45-2.43 (m, 2H), 1.89-1.68 (m, 3H), 1.51-1.48 (m, 3H), 1.33-1.23 (m, 3H), 0.94-0.75 (m, 9H);
ESI-MS m/z =359.2, [M+Na]⁺.

### Example 9: Synthesis of Compound N9(S)

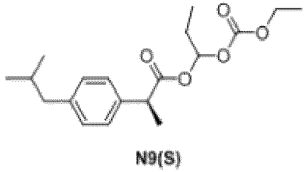

The operations were the same as those in Example 8 except that ibuprofen was replaced with S-ibuprofen.
¹H NMR (400 MHz, CDCl₃) δ 7.19-7.07 (m, 4H), 6.65-6.59 (m, 1H), 4.24-4.09 (m, 2H), 3.73-3.68 (m, 1H), 2.45-2.43 (m, 2H), 1.89-1.68 (m, 3H), 1.51-1.48 (m, 3H), 1.33-1.23 (m, 3H), 0.94-0.75 (m, 9H);
ESI-MS m/z =359.2, [M+Na]⁺.

### Example 10: Synthesis of Compound N10

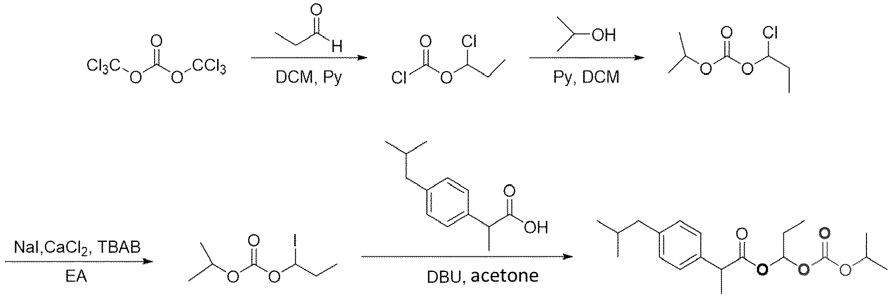

**Synthesis of 1-chloropropyl chloroformate:** triphosgene (10 g, 33.70 mmol) was weighed into a 100 mL three-necked reaction flask and added with 15 mL of anhydrous dichloromethane, and the reaction flask was purged with Ar three times, transferred to a cold trap of -20 °C, and stirred continuously. Py (0.54 g, 6.83 mmol) was diluted with 5 mL of dichloromethane and then added to the reaction flask. N-propionaldehyde (4.09 g, 70.42 mmol) was weighed and slowly added dropwise to the reaction flask, and after the drop, the temperature of the cold trap was set to -2 °C and the reaction was continued for 20 h. The reaction system was subjected to gas extraction for 5 min with a water pump that was connected with a flask containing an aqueous solution of KOH, and then transferred out of the cold trap. The solution was concentrated under reduced pressure for DCM to be removed and then distilled to obtain 6.44 g of a colorless oil with a yield of 58.6%.

**Synthesis of 1-chloropropyl isopropyl carbonate:** 1-chloropropyl chloroformate (1.0 g, 6.41 mmol) was weighed into a dry two-necked reaction flask, added with 10 mL of anhydrous DCM, and stirred continuously. Isopropanol (0.578 g, 9.62 mmol) was weighed into the above reaction flask. The reaction flask was transferred to an ice-water bath and stirred continuously. Pyridine (0.623 g, 7.88 mmol) was weighed and slowly added dropwise to the above reaction flask with a white solid appearing, and after the drop, the reaction flask was transferred to room temperature for a reaction of 1 h. 10 mL of water was added to the reaction flask, layers were separated, and the DCM layer was washed with 5% KHSO₄ to a pH of 3-4, washed with water to nearly neutral, washed with saturated salt solution, and dried over anhydrous sodium sulfate. The resulting product was concentrated under reduced pressure to obtain 0.810 g of a colorless oil, which was directly used in the next step without purification.

**Synthesis of 1-iodopropyl isopropyl carbonate:** 1-chloropropyl isopropyl carbonate (0.810 g, 4.50 mmol), CaCl₂ (0.174 g, 1.57 mmol), TBAB (0.027 g, 0.09 mmol), and NaI (0.570 g, 3.80 mmol) were weighed into a 100 mL two-necked reaction flask, added with 50 mL of ethyl acetate, and heated to reflux for 3 h. 20 mL of water was added to the reaction flask, layers were separated, and the EA layer was washed twice with 5% sodium thiosulfate, washed with saturated salt solution, dried over anhydrous sodium sulfate, and concentrated to obtain a yellow oil, which was directly used in the next step without purification.

**Synthesis of N10:** ibuprofen (0.464 g, 2.25 mmol) was weighed into a dry single-necked reaction flask, added with 10 mL of acetone, and stirred to be dissolved, 1-iodopropyl isopropyl carbonate (0.920 g, 3.38 mmol) was added, and DBU (0.309 g, 2.03 mmol) was slowly added dropwise under an ice bath. The reaction was conducted for 3 h at 40 °C. DCM and water were added to the reaction, layers were separated, and the DCM layer was washed with 5% NaHCO₃, water, and saturated salt solution, dried, and concentrated. The resulting product was subjected to flash column chromatography (PE/EA = 60:1) to obtain 0.50 g of a colorless oil with a yield of 63.5%.
¹H NMR (400 MHz, CDCl₃) δ 7.20-7.17 (m, 2H), 7.09-7.06 (m, 2H), 6.66-6.59 (m, 1H), 4.98-4.85 (m, 1H), 3.702 (q, *J* =7.1 Hz, 1H), 2.4335 (dd, *J* =3.3, 7.2 Hz, 2H), 1.87-1.67 (m, 3H), 1.494 (dd, *J* =1.9, 7.2 Hz, 3H), 1.314-1.207 (m, 6H), 0.941-0.902, 0.786-0.748 (m, 3H), 0.896-0.871 (m, 3H);
ESI-MS m/z =373.2, [M+Na]⁺.

### Example 11: Synthesis of Compound N10(S)

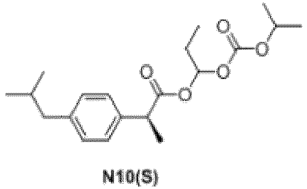

The operations were the same as those in Example 10 except that ibuprofen was replaced with S-ibuprofen.
¹H NMR (400 MHz, CDCl₃) δ 7.20-7.17 (m, 2H), 7.09-7.06 (m, 2H), 6.66-6.59 (m, 1H), 4.98-4.85 (m, 1H), 3.702 (q, *J* =7.1 Hz, 1H), 2.4335 (dd, *J* =3.3, 7.2 Hz, 2H), 1.87-1.67 (m, 3H), 1.494 (dd, *J* =1.9, 7.2 Hz, 3H), 1.314-1.207 (m, 6H), 0.941-0.902, 0.786-0.748 (m, 3H), 0.896-0.871 (m, 3H);
ESI-MS m/z =373.2, [M+Na]⁺.

### Example 12: Synthesis of Compound N11

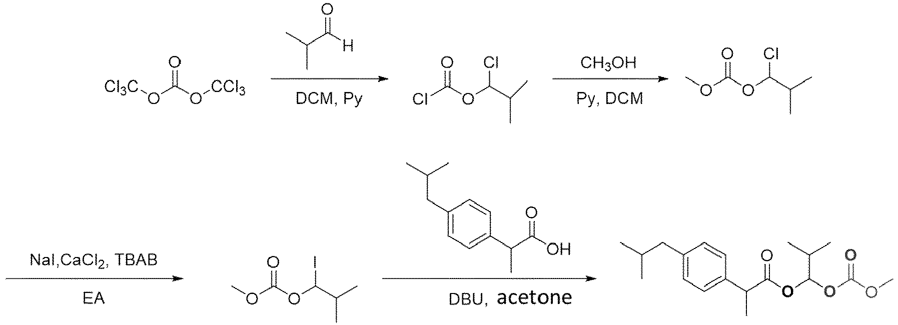

**Synthesis of 1-chloroisobutyl chloroformate:** triphosgene (20 g, 67.40 mmol) was weighed into a 100 mL three-necked reaction flask and added with 25 mL of anhydrous dichloromethane, and the reaction flask was purged with Ar three times, transferred to a cold trap of -20 °C, and stirred continuously. Py (1.10 g, 13.80 mmol) was diluted with 5 mL of dichloromethane and then added to the reaction flask. Isobutyraldehyde (10.170 g, 141.05 mmol) was weighed and slowly added dropwise to the reaction flask, and after the drop, the temperature of the cold trap was set to -2 °C and the reaction was continued for 20 h. The reaction system was subjected to gas extraction for 5 min with a water pump that was connected with a flask containing an aqueous solution of KOH, and then transferred out of the cold trap. The solution was concentrated under reduced pressure for DCM to be removed and then distilled to obtain 9.55 g of a colorless oil with a yield of 39.8%.

**Synthesis of 1-chloroisobutyl methyl carbonate:** 1-chloroisobutyl chloroformate (1.0 g, 6.02 mmol) was weighed into a dry two-necked reaction flask, added with 10 mL of anhydrous DCM, and stirred continuously. Methanol (0.293 g, 9.15 mmol) was weighed into the above reaction flask. The reaction flask was transferred to an ice-water bath and stirred continuously. Pyridine (0.578 g, 7.31 mmol) was weighed and slowly added dropwise to the above reaction flask with a white solid appearing, and after the drop, the reaction flask was transferred to room temperature for a reaction of 1 h. 10 mL of water was added to the reaction flask, layers were separated, and the DCM layer was washed with 5% KHSO₄ to a pH of 3-4, washed with water to nearly neutral, washed with saturated salt solution, and dried over anhydrous sodium sulfate. The resulting product was concentrated under reduced pressure to obtain 0.747 g of a colorless oil, which was directly used in the next step without purification.

**Synthesis of 1-iodoisobutyl methyl carbonate:** 1-chloroisobutyl methyl carbonate (0.747 g, 4.50 mmol), CaCl₂ (0.174 g, 1.57 mmol), TBAB (0.027 g, 0.09 mmol), and NaI (0.570 g, 3.80 mmol) were weighed into a 100 mL two-necked reaction flask, added with 50 mL of ethyl acetate, and heated to reflux for 3 h. 20 mL of water was added to the reaction flask, layers were separated, and the EA layer was washed twice with 5% sodium thiosulfate, washed with saturated salt solution, dried over anhydrous sodium sulfate, and concentrated to obtain a yellow oil, which was directly used in the next step without purification.

**Synthesis of N11**: ibuprofen (0.450 g, 2.18 mmol) was weighed into a dry single-necked reaction flask, added with 10 mL of acetone, and stirred to be dissolved, 1-iodoisobutyl methyl carbonate (0.880 g, 3.41 mmol) was added, and DBU (0.304 g, 2.00 mmol) was slowly added dropwise under an ice bath. The reaction was conducted for 3 h at 40 °C. DCM and water were added to the reaction, layers were separated, and the DCM layer was washed with 5% NaHCO₃, water, and saturated salt solution, dried, and concentrated. The resulting product was subjected to flash column chromatography (PE/EA = 60:1) to obtain 0.57 g of a colorless oil with a yield of 78.0%.
¹H NMR (400 MHz, CDCl₃) δ 7.19-7.07 (m, 4H), 6.65-6.59 (m, 1H), 4.24-4.09 (d, *J* =29.2 Hz, 3H), 3.73-3.68 (m, 1H), 2.45-2.43 (m, 2H), 1.89-1.68 (m, 2H), 1.51-1.48 (m, 3H), 1.33-1.23 (m, 3H), 0.94-0.75 (m, 9H);
ESI-MS m/z =359.2, [M+Na]⁺.

### Example 13: Synthesis of Compound N12

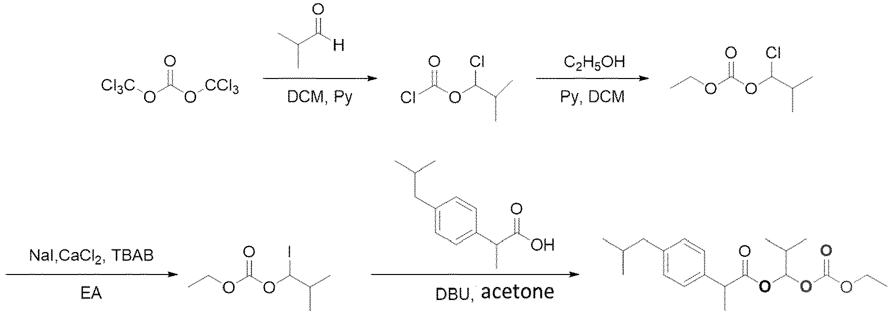

**Synthesis of 1-chloroisobutyl chloroformate:** triphosgene (20 g, 67.40 mmol) was weighed into a 100 mL three-necked reaction flask and added with 25 mL of anhydrous dichloromethane, and the reaction flask was purged with Ar three times, transferred to a cold trap of -20 °C, and stirred continuously. Py (1.10 g, 13.80 mmol) was diluted with 5 mL of dichloromethane and then added to the reaction flask. Isobutyraldehyde (10.170 g, 141.05 mmol) was weighed and slowly added dropwise to the reaction flask, and after the drop, the temperature of the cold trap was set to -2 °C and the reaction was continued for 20 h. The reaction system was subjected to gas extraction for 5 min with a water pump that was connected with a flask containing an aqueous solution of KOH, and then transferred out of the cold trap. The solution was concentrated under reduced pressure for DCM to be removed and then distilled to obtain 9.55 g of a colorless oil with a yield of 39.8%.

**Synthesis of 1-chloroisobutyl ethyl carbonate:** 1-chloroisobutyl chloroformate (2.0 g, 12.04 mmol) was weighed into a dry two-necked reaction flask, added with 10 mL of anhydrous DCM, and stirred continuously. Ethanol (0.843 g, 18.30 mmol) was weighed into the above reaction flask. The reaction flask was transferred to an ice-water bath and stirred continuously. Pyridine (1.132 g, 14.31 mmol) was weighed and slowly added dropwise to the above reaction flask with a white solid appearing, and after the drop, the reaction flask was transferred to room temperature for a reaction of 1 h. 10 mL of water was added to the reaction flask, layers were separated, and the DCM layer was washed with 5% KHSO₄ to a pH of 3-4, washed with water to nearly neutral, washed with saturated salt solution, and dried over anhydrous sodium sulfate. The resulting product was concentrated under reduced pressure to obtain 1.512 g of a colorless oil, which was directly used in the next step without purification.

**Synthesis of 1-iodoisobutyl ethyl carbonate:** 1-chloroisobutyl ethyl carbonate (1.512 g, 8.40 mmol), CaCl₂ (0.317 g, 2.86 mmol), TBAB (0.054 g, 0.17 mmol), and NaI (1.058 g, 7.06 mmol) were weighed into a 100 mL two-necked reaction flask, added with 50 mL of ethyl acetate, and heated to reflux for 3 h. 20 mL of water was added to the reaction flask, layers were separated, and the EA layer was washed twice with 5% sodium thiosulfate, washed with saturated salt solution, dried over anhydrous sodium sulfate, and concentrated to obtain a yellow oil, which was directly used in the next step without purification.

**Synthesis of N12:** ibuprofen (1.237 g, 6.00 mmol) was weighed into a dry single-necked reaction flask, added with 10 mL of acetone, and stirred to be dissolved, 1-iodoisobutyl ethyl carbonate (2.478 g, 9.11 mmol) was added, and DBU (0.837 g, 5.50 mmol) was slowly added dropwise under an ice bath. The reaction was conducted for 3 h at 40 °C. DCM and water were added to the reaction, layers were separated, and the DCM layer was washed with 5% NaHCO₃, water, and saturated salt solution, dried, and concentrated. The resulting product was subjected to flash column chromatography (PE/EA = 60:1) to obtain 1.30 g of a colorless oil with a yield of 61.9%.
¹H NMR (400 MHz, CDCl₃) δ 7.19-7.07 (m, 4H), 6.65-6.59 (m, 1H), 4.24-4.09 (m, 2H), 3.73-3.68 (m, 1H), 2.45-2.43 (m, 2H), 1.89-1.68 (m, 2H), 1.51-1.48 (m, 3H),1.33-1.23 (m, 6H), 0.94-0.75 (m, 9H);
ESI-MS m/z =373.2, [M+Na]⁺.

### Example 14: Synthesis of Compound N13

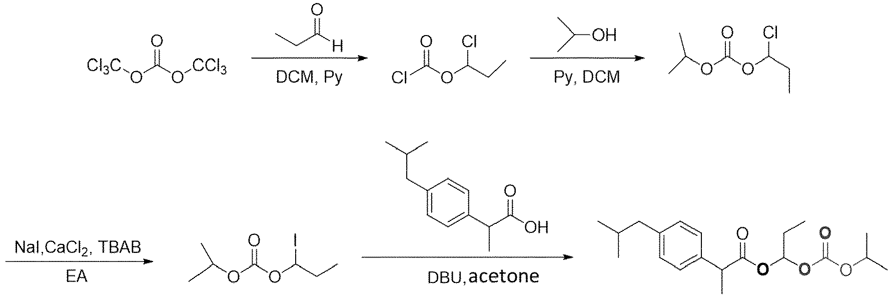

**Synthesis of 1-chloropropyl chloroformate:** triphosgene (10 g, 33.70 mmol) was weighed into a 100 mL three-necked reaction flask and added with 15 mL of anhydrous dichloromethane, and the reaction flask was purged with Ar three times, transferred to a cold trap of -20 °C, and stirred continuously. Py (0.54 g, 6.83 mmol) was diluted with 5 mL of dichloromethane and then added to the reaction flask. N-propionaldehyde (4.09 g, 70.42 mmol) was weighed and slowly added dropwise to the reaction flask, and after the drop, the temperature of the cold trap was set to -2 °C and the reaction was continued for 20 h. The reaction system was subjected to gas extraction for 5 min with a water pump that was connected with a flask containing an aqueous solution of KOH, and then transferred out of the cold trap. The solution was concentrated under reduced pressure for DCM to be removed and then distilled to obtain 6.44 g of a colorless oil with a yield of 58.6%.

**Synthesis of 1-chloropropyl isopropyl carbonate:** 1-chloropropyl chloroformate (1.0 g, 6.41 mmol) was weighed into a dry two-necked reaction flask, added with 10 mL of anhydrous DCM, and stirred continuously. Isopropanol (0.578 g, 9.62 mmol) was weighed into the above reaction flask. The reaction flask was transferred to an ice-water bath and stirred continuously. Pyridine (0.623 g, 7.88 mmol) was weighed and slowly added dropwise to the above reaction flask with a white solid appearing, and after the drop, the reaction flask was transferred to room temperature for a reaction of 1 h. 10 mL of water was added to the reaction flask, layers were separated, and the DCM layer was washed with 5% KHSO₄ to a pH of 3-4, washed with water to nearly neutral, washed with saturated salt solution, and dried over anhydrous sodium sulfate. The resulting product was concentrated under reduced pressure to obtain 0.810 g of a colorless oil, which was directly used in the next step without purification.

**Synthesis of 1-iodopropyl isopropyl carbonate:** 1-chloropropyl isopropyl carbonate (0.810 g, 4.50 mmol), CaCl₂ (0.174 g, 1.57 mmol), TBAB (0.027 g, 0.09 mmol), and NaI (0.570 g, 3.80 mmol) were weighed into a 100 mL two-necked reaction flask, added with 50 mL of ethyl acetate, and heated to reflux for 3 h. 20 mL of water was added to the reaction flask, layers were separated, and the EA layer was washed twice with 5% sodium thiosulfate, washed with saturated salt solution, dried over anhydrous sodium sulfate, and concentrated to obtain a yellow oil, which was directly used in the next step without purification.

**Synthesis of N13:** ibuprofen (0.464 g, 2.25 mmol) was weighed into a dry single-necked reaction flask, added with 10 mL of acetone, and stirred to be dissolved, 1-iodopropyl isopropyl carbonate (0.920 g, 3.38 mmol) was added, and DBU (0.309 g, 2.03 mmol) was slowly added dropwise under an ice bath. The reaction was conducted for 3 h at 40 °C. DCM and water were added to the reaction, layers were separated, and the DCM layer was washed with 5% NaHCO₃, water, and saturated salt solution, dried, and concentrated. The resulting product was subjected to flash column chromatography (PE/EA = 60:1) to obtain 0.50 g of a colorless oil with a yield of 63.5%.
¹H NMR (400 MHz, CDCl₃) δ 7.20-7.17 (m, 2H), 7.09-7.06 (m, 2H), 6.66-6.59 (m, 1H), 4.98-4.85 (m, 1H), 3.702 (q, *J* =7.1 Hz, 1H), 2.4335 (dd, *J* =3.3, 7.2 Hz, 2H), 1.87-1.67 (m, 3H), 1.494 (dd, *J* =1.9, 7.2 Hz, 3H), 1.314-1.207 (m, 6H), 0.941-0.902, 0.786-0.748 (m, 3H), 0.896-0.871 (m, 3H);
ESI-MS m/z =373.2, [M+Na]⁺.

### Example 15: Synthesis of Compound N14

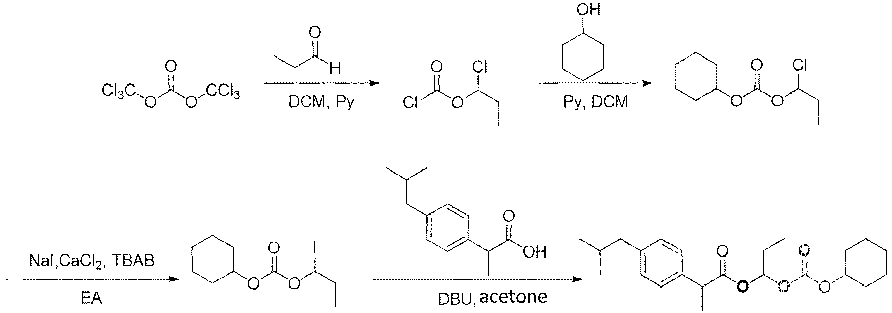

**Synthesis of 1-chloropropyl chloroformate:** triphosgene (10 g, 33.70 mmol) was weighed into a 100 mL three-necked reaction flask and added with 15 mL of anhydrous dichloromethane, and the reaction flask was purged with Ar three times, transferred to a cold trap of -20 °C, and stirred continuously. Py (0.54 g, 6.83 mmol) was diluted with 5 mL of dichloromethane and then added to the reaction flask. N-propionaldehyde (4.09 g, 70.42 mmol) was weighed and slowly added dropwise to the reaction flask, and after the drop, the temperature of the cold trap was set to -2 °C and the reaction was continued for 20 h. The reaction system was subjected to gas extraction for 5 min with a water pump that was connected with a flask containing an aqueous solution of KOH, and then transferred out of the cold trap. The solution was concentrated under reduced pressure for DCM to be removed and then distilled to obtain 6.44 g of a colorless oil with a yield of 58.6%.

**Synthesis of 1-chloropropyl cyclohexyl carbonate:** 1-chloropropyl chloroformate (1.0 g, 6.41 mmol) was weighed into a dry two-necked reaction flask, added with 10 mL of anhydrous DCM, and stirred continuously. Alcohol (0.921 g, 9.20 mmol) was weighed into the above reaction flask. The reaction flask was transferred to an ice-water bath and stirred continuously. Pyridine (0.609 g, 7.70 mmol) was weighed and slowly added dropwise to the above reaction flask with a white solid appearing, and after the drop, the reaction flask was transferred to room temperature for a reaction of 1 h. 10 mL of water was added to the reaction flask, layers were separated, and the DCM layer was washed with 5% KHSO₄ to a pH of 3-4, washed with water to nearly neutral, washed with saturated salt solution, and dried over anhydrous sodium sulfate. The resulting product was concentrated under reduced pressure to obtain 0.836 g of a colorless oil, which was directly used in the next step without purification.

**Synthesis of 1-iodopropyl cyclohexyl carbonate:** 1-chloropropyl cyclohexyl carbonate (0.836 g, 3.80 mmol), CaCl₂ (0.184 g, 1.66 mmol), TBAB (0.031 g, 0.10 mmol), and NaI (0.480 g, 3.20 mmol) were weighed into a 100 mL two-necked reaction flask, added with 50 mL of ethyl acetate, and heated to reflux for 3 h. 20 mL of water was added to the reaction flask, layers were separated, and the EA layer was washed twice with 5% sodium thiosulfate, washed with saturated salt solution, dried over anhydrous sodium sulfate, and concentrated to obtain a yellow oil, which was directly used in the next step without purification.

**Synthesis of N14:** ibuprofen (0.392 g, 1.90 mmol) was weighed into a dry single-necked reaction flask, added with 10 mL of acetone, and stirred to be dissolved, 1-iodopropyl cyclohexyl carbonate (0.880 g, 2.82 mmol) was added, and DBU (0.266 g, 1.75 mmol) was slowly added dropwise under the condition of an ice bath. The reaction was conducted for 3 h at 40 °C. DCM and water were added to the reaction, layers were separated, and the DCM layer was washed with 5% NaHCO₃, water, and saturated salt solution, dried, and concentrated. The resulting product was subjected to flash column chromatography (PE/EA = 60:1) to obtain 0.49 g of a colorless oil with a yield of 66.1%.
¹H NMR (400 MHz, CDCl₃) δ 7.20-7.06 (m, 4H), 6.66-6.59 (m, 1H), 4.66-4.48 (m, 1H), 3.73-3.67 (m, 1H), 2.45-2.42 (m, 2H), 1.87-1.69 (m, 5H), 1.51-1.20 (m, 11H), 0.95-0.90, 0.79-0.74 (m, 3H), 0.893 (d, *J* =3**.**3*,* 3H), 0.876 (d, *J* =3.3 Hz, 3H);
ESI-MS m/z =413.2, [M+Na]⁺.

### Example 16: Synthesis of Compound N15

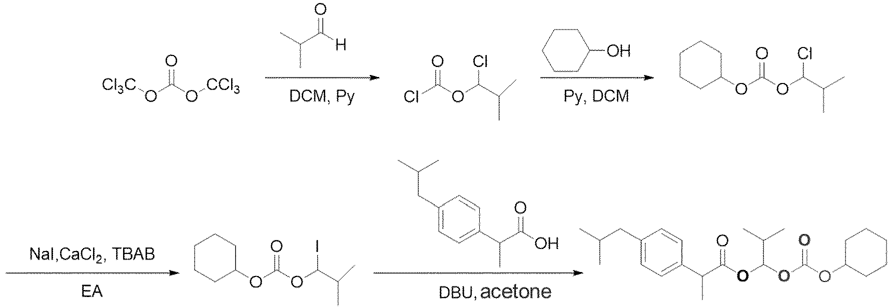

**Synthesis of 1-chloroisobutyl chloroformate:** triphosgene (20 g, 67.40 mmol) was weighed into a 100 mL three-necked reaction flask and added with 25 mL of anhydrous dichloromethane, and the reaction flask was purged with Ar three times, transferred to a cold trap of -20 °C, and stirred continuously. Py (1.10 g, 13.80 mmol) was diluted with 5 mL of dichloromethane and then added to the reaction flask. Isobutyraldehyde (10.170 g, 141.05 mmol) was weighed and slowly added dropwise to the reaction flask, and after the drop, the temperature of the cold trap was set to -2 °C and the reaction was continued for 20 h. The reaction system was subjected to gas extraction for 5 min with a water pump that was connected with a flask containing an aqueous solution of KOH, and then transferred out of the cold trap. The solution was concentrated under reduced pressure for DCM to be removed and then distilled to obtain 9.55 g of a colorless oil with a yield of 39.8%.

**Synthesis of 1-chloroisobutyl cyclohexyl carbonate:** 1-chloroisobutyl chloroformate (2.0 g, 12.04 mmol) was weighed into a dry two-necked reaction flask, added with 10 mL of anhydrous DCM, and stirred continuously. Cyclohexanol (1.803 g, 18.00 mmol) was weighed into the above reaction flask. The reaction flask was transferred to an ice-water bath and stirred continuously. Pyridine (1.125 g, 14.22 mmol) was weighed and slowly added dropwise to the above reaction flask with a white solid appearing, and after the drop, the reaction flask was transferred to room temperature for a reaction of 1 h. 10 mL of water was added to the reaction flask, layers were separated, and the DCM layer was washed with 5% KHSO₄ to a pH of 3-4, washed with water to nearly neutral, washed with saturated salt solution, and dried over anhydrous sodium sulfate. The resulting product was concentrated under reduced pressure to obtain 1.710 g of a colorless oil, which was directly used in the next step without purification.

**Synthesis of 1-iodoisobutyl isopropyl carbonate:** 1-chloroisobutyl isopropyl carbonate (1.710 g, 7.30 mmol), CaCl₂ (0.276 g, 2.48 mmol), TBAB (0.048 g, 0.15 mmol), and NaI (1.003 g, 6.69 mmol) were weighed into a 100 mL two-necked reaction flask, added with 50 mL of ethyl acetate, and heated to reflux for 3 h. 20 mL of water was added to the reaction flask, layers were separated, and the EA layer was washed twice with 5% sodium thiosulfate, washed with saturated salt solution, dried over anhydrous sodium sulfate, and concentrated to obtain a yellow oil, which was directly used in the next step without purification.

**Synthesis of N15:** ibuprofen (0.680 g, 3.30 mmol) was weighed into a dry single-necked reaction flask, added with 10 mL of acetone, and stirred to be dissolved, 1-iodoisobutyl ethyl carbonate (1.612 g, 4.94 mmol) was added, and DBU (0.458 g, 3.01 mmol) was slowly added dropwise under an ice bath. The reaction was conducted for 3 h at 40 °C. DCM and water were added to the reaction, layers were separated, and the DCM layer was washed with 5% NaHCO₃, water, and saturated salt solution, dried, and concentrated. The resulting product was subjected to flash column chromatography (PE/EA = 60:1) to obtain 0.66 g of a colorless oil with a yield of 49.5%.
¹H NMR (400 MHz, CDCl₃) δ 7.20-7.06 (m, 4H), 6.66-6.59 (m, 1H), 4.66-4.48 (m, 1H), 3.73-3.67 (m, 1H), 2.45-2.42 (m, 2H), 1.87-1.69 (m, 4H), 1.51-1.20 (m, 11H), 0.95-0.90, 0.79-0.74 (m, 3H), 0.893 (m, 6H), 0.876 (m, 6H);
ESI-MS m/z =427.2, [M+Na]⁺.

### Example 17: Synthesis of Compound N18

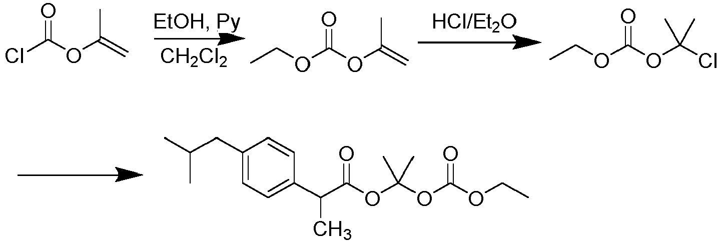

**Synthesis of 1-chloro-1-methyl-ethyl ethyl carbonate:** 2-propenyl chloroformate (1.00 g, 8.30 mmol) was weighed into a single-necked reaction flask, added with 20 mL of anhydrous dichloromethane, and stirred continuously. Ethanol (0.38 g, 8.30 mmol) was weighed into the above reaction flask, and the reaction flask was transferred to an ice bath and stirred. Pyridine (0.69 g, 8.72 mmol) diluted with 5 mL of dry CH₂Cl₂ was slowly added dropwise and then reacted at room temperature for 2 h. The system was poured into 20 mL of ice water, an organic phase was dried over anhydrous sodium sulfate and filtered, and the filtrate was evaporated to dryness to obtain an oily liquid. The liquid was added with 10 mL of ether and then 50 mL of 4N hydrochloric acid/ether was slowly added dropwise, stirred overnight at room temperature, and concentrated under reduced pressure to obtain 1.1 g of a colorless liquid, which was directly used in the next step without purification.

**Synthesis of Compound N18:** ibuprofen (0.91g, 4.4 mmol) was weighed at room temperature into a dry 50 mL single-necked reaction flask, added with 10 mL of acetone, and stirred to be dissolved. Then, 1-chloro-1-methyl-ethyl ethyl carbonate (1.1 g, 6.62 mmol) was weighed and added to the above reaction flask, and DBU (0.672 g, 4.4 mmol) was weighed and slowly added dropwise to the reaction flask. The reaction was stirred at room temperature overnight after the drop. Water (10 mL) and ethyl acetate (30 mL) were added to the reaction flask, layers were separated, and the organic layer was washed with 5% NaHCO₃, water, and saturated salt solution, dried over anhydrous sodium sulfate, concentrated under reduced pressure, and subjected to flash column chromatography (PE/EA = 4:1) to obtain 1.05 g of a target product with a yield of 71%.
¹H NMR (400 MHz, CDCl₃) δ 7.19-7.07 (m, 4H), 4.24-4.08 (m, 2H), 3.72-3.66 (m, 1H), 2.44 (d, 2H, *J =* 7.1 Hz), 1.89-1.79 (m, 1H), 1.60-1.34 (m, 9H), 1.33-1.22 (m,3H), 0.89 (d, 6H, *J* = 6.6 Hz);
ESI-MS m/z =337.2, [M+H]⁺.

### Example 18: Synthesis of Compound N18(S)

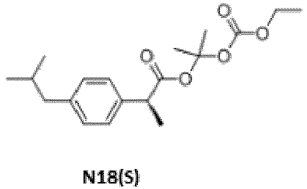

The operations were the same as those in Example 17 except that ibuprofen was replaced with S-ibuprofen.
¹H NMR (400 MHz, CDCl₃) δ 7.19-7.07 (m, 4H), 4.24-4.08 (m, 2H), 3.72-3.66 (m, 1H), 2.44 (d, 2H, *J =* 7.1 Hz), 1.89-1.79 (m, 1H), 1.60-1.34 (m, 9H), 1.33-1.22 (m, 3H), 0.89 (d, 6H, *J* = 6.6 Hz);
ESI-MS m/z =337.2, [M+H]⁺.

### Example 19: Synthesis of Compound N19

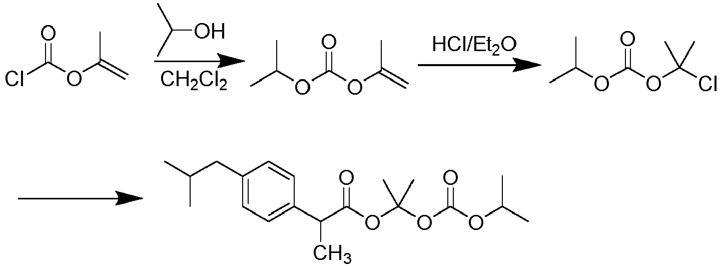

**Synthesis of 1-chloro-1-methyl-ethyl isopropyl carbonate:** 2-propenyl chloroformate (1.00 g, 8.30 mmol) was weighed into a single-necked reaction flask, added with 20 mL of anhydrous dichloromethane, and stirred continuously. Isopropanol (0.50 g, 8.30 mmol) was weighed into the above reaction flask, and the reaction flask was transferred to an ice bath and stirred. Pyridine (0.69 g, 8.72 mmol) diluted with 5 mL of dry CH₂Cl₂ was slowly added dropwise and then reacted at room temperature for 2 h. The system was poured into 20 mL of ice water, an organic phase was dried over anhydrous sodium sulfate and filtered, and the filtrate was evaporated to dryness to obtain an oily liquid. The liquid was added with 10 mL of ether and then 50 mL of 4N hydrochloric acid/ether was slowly added dropwise, stirred overnight at room temperature, and concentrated under reduced pressure to obtain 0.94 g of a colorless liquid, which was directly used in the next step without purification.

**Synthesis of Compound N19:** ibuprofen (0.5 g, 2.42 mmol) was weighed at room temperature into a dry 50 mL single-necked reaction flask, added with 10 mL of acetone, and stirred to be dissolved. Then, 1-chloro-1-methyl-ethyl isopropyl carbonate (0.94 g, 5.52 mmol) was weighed and added to the above reaction flask, and DBU (0.37 g, 2.42 mmol) was weighed and slowly added dropwise to the reaction flask. The reaction was stirred at room temperature overnight after the drop. Water (10 mL) and ethyl acetate (30 mL) were added to the reaction flask, layers were separated, and the organic layer was washed with 5% NaHCO₃, water, and saturated salt solution, dried over anhydrous sodium sulfate, concentrated under reduced pressure, and subjected to flash column chromatography (PE/EA = 4:1) to obtain 0.52 g of a target product with a yield of 52%.
¹H NMR (400 MHz, CDCl₃) δ 7.19-7.06 (m, 4H), 4.91-4.75 (m, 1H), 3.72-3.66 (m, 1H), 2.45-2.42 (m, 2H), 1.91-1.85 (m, 1H), 1.69-1.49 (m, 9H), 1.36-1.20 (m, 6H), 0.889 (d, *J =* 6.6 Hz, 6H);
ESI-MS m/z =351.2, [M+H]⁺.

### Example 20: Synthesis of Compound N19(S)

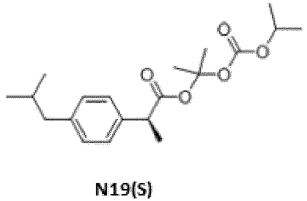

The operations were the same as those in Example 19 except that ibuprofen was replaced with S-ibuprofen.
¹H NMR (400 MHz, CDCl₃) δ 7.19-7.06 (m, 4H), 4.91-4.75 (m, 1H), 3.72-3.66 (m, 1H), 2.45-2.42 (m, 2H), 1.91-1.85 (m, 1H), 1.69-1.49 (m, 9H), 1.36-1.20 (m, 6H), 0.889 *(d, J* = 6.6 Hz, 6H);
ESI-MS m/z =351.2, [M+H]⁺.

### Comparative Example 1

A commercially available drug, flurbiprofen axetil (FPA), was purchased as a reference compound for the compounds of the present application.

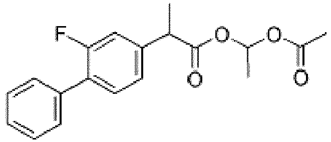

### Test Example 1: Study on the stability of the compound at a high temperature

Test scheme: An appropriate amount of the compound prepared in the present application was put in a vial and placed at a high temperature (60 °C) with light excluded. Samples were taken on Day 0, 5 and 10 to investigate the purity of the compound and changes of the related substance (ibuprofen). The results are shown in Table 1.

**Table 1 Purity of the compound of the present application after placed at a high temperature (60 °C) with light excluded and the related substance (ibuprofen)**

| | | Day 0 | Day 5 | Day 10 |
|---|---|---|---|---|
| N2 | Total purity of main peak (%) | 99.68 | 99.58 | 98.59 |
| | Ibuprofen (%) | / | / | / |
| N4 | Total purity of main peak (%) | 98.43 | 98.26 | 97.56 |
| | Ibuprofen (%) | / | 0.06 | 0.10 |
| N6 | Total purity of main peak (%) | 99.81 | 99.77 | 98.81 |
| | Ibuprofen (%) | 0.06 | 0.06 | 0.06 |
| N9 | Total purity of main peak (%) | 99.56 | 99.53 | 98.30 |
| | Ibuprofen (%) | / | / | 0.01 |
| N10 | Total purity of main peak (%) | 99.36 | 99.19 | 94.84 |
| | Ibuprofen (%) | / | 0.02 | 0.04 |
| N12 | Total purity of main peak (%) | 95.86 | 95.74 | 92.05 |
| | Ibuprofen (%) | / | / | 0.19 |
| N13 | Total purity of main peak (%) | 98.24 | 97.16 | 96.13 |
| | Ibuprofen (%) | 0.04 | 0.06 | 0.10 |
| N14 | Total purity of main peak (%) | 98.87 | 98.75 | 97.47 |
| | Ibuprofen (%) | / | / | 0.01 |
| N18 | Total purity of main peak (%) | 99.63 | 99.32 | 98.25 |
| | Ibuprofen (%) | / | / | 0.02 |
| N19 | Total purity of main peak (%) | 99.57 | 99.46 | 98.34 |
| | Ibuprofen (%) | / | / | 0.01 |

As can be seen from the test results, the compounds of the present application all have a purity of greater than 92% and most compounds have a purity of greater than 95% on Day 10 after placement at a high temperature (60 °C) with light excluded, indicating good stability.

### Test Example 2 Toxicity of by-products

According to the metabolic pathways of the compounds in the human body, the metabolic by-products of Compounds N2 to N19 in the human body and their toxicity are shown in Table 2.

**Table 2**

| No. | Metabolic By-product in the Human Body | Toxicity |
|---|---|---|
| N2 | Acetaldehyde, isopropanol | Low toxicity |
| N4 | Acetaldehyde, cyclohexanol | Low toxicity |
| N6 | Acetaldehyde, ethanol | Low toxicity |
| N7 | Acetaldehyde, methanol | Relatively high toxicity of methanol |
| N8 | Propionaldehyde, methanol | Relatively high toxicity of methanol |
| N9 | Propionaldehyde, ethanol | Low toxicity |
| N10 | Propionaldehyde, isopropanol | Low toxicity |
| N11 | Iso-butyraldehyde, methanol | Relatively high toxicity of methanol |
| N12 | Iso-butyraldehyde, ethanol | Relatively high toxicity of iso-butyraldehyde |
| N13 | Iso-butyraldehyde, isopropanol | Relatively high toxicity of iso-butyraldehyde |
| N14 | Propionaldehyde, cyclohexanol | Low toxicity |
| N15 | Iso-butyraldehyde, cyclohexanol | Relatively high toxicity of iso-butyraldehyde |
| N18 | Acetone, ethanol | Low toxicity |
| N19 | Acetone, isopropanol | Low toxicity |

As can be seen from the test results, this series of compounds produce by-products with relatively low toxicity after metabolism in the human body, especially Compounds N2, N4, N6, N9, N10, N14, N18, and N19 have good clinical application prospects.

Compounds N2, N6, N9, N10, N18, N19 and their dextrorotatory enantiomers (that is, isomers whose carbon at position 1 is in an S configuration) have not only by-products with low toxicity after metabolism in the human body, but also a faster metabolic rate, which are more suitable for development into injection preparations. The mode of administration may be selected from subcutaneous injection, intramuscular injection, intravenous injection, intravenous infusion, or the like.

In addition, Compounds N4, N14 and their dextrorotatory enantiomers (that is, isomers whose carbon at position 1 is in an S configuration) have not only by-products with low toxicity after metabolism in the human body, but also a slow metabolic rate, which are more suitable for development into long-acting sustained-release preparations.

### Test Example 3: Study on metabolism of the compound of the present application in human plasma

A prodrug exerts efficacy through a drug released through the enzymolysis of the prodrug in a body. Therefore, the metabolic rate and production rate of the prodrug in plasma are closely related to whether the prodrug can effectively exert efficacy and prolong a half-life. In the present application, the conversion characteristics of the preceding preferred compounds, the reference compound, and their isomers were evaluated by establishing an in vitro human plasma metabolism model. The test scheme was as follows.

3.1 Compounds NX prepared in the examples and flurbiprofen axetil in Comparative Example 1 were subjected to an enzymolysis kinetics test in human plasma in batches. The test method was as follows:
(1) a stock solution of Compound NX (where X represents a different compound number) of the present application in pure acetonitrile (40 mM) was prepared, a stock solution of flurbiprofen axetil in pure acetonitrile (40 mM) was prepared, and a stock solution of ibuprofen in pure acetonitrile (40 mM) was prepared;
(2) the stock solution of flurbiprofen axetil/ibuprofen (25 µL) was mixed with 1 mL of human plasma and vortexed for 30s. A sample (200 µL) was taken, added with 800 µL of acetonitrile to precipitate proteins and vortexed for 1 min, and the reaction was stopped as a flurbiprofen axetil/ibuprofen control; and the stock solution of the compound of the present application (40 mM) and the stock solution of flurbiprofen axetil (40 mM) were diluted 200 times separately as prodrug controls;
(4) the stock solution of the compound of the present application in pure acetonitrile (100 µL) and the stock solution of flurbiprofen axetil in pure acetonitrile (100 µL) were mixed with 4 mL of human plasma, separately, vortexed for 30s, and placed in an oscillating constant temperature water bath heater at 37 °C and oscillated at 200 rpm;
(5) samples (200 µL) were taken at different time points (0, 15, 30, 60 and 120 min) with three samples at each time point, added with 800 µL of acetonitrile to precipitate proteins, and vortexed for 1 min, and then the reaction was stropped; and a blank plasma control was obtained in the same manner;
(6) the samples were centrifuged at 12000 rpm for 10 min at 4 °C, the supernatant was taken and injected at a volume of 30 µL (through a filter membrane), and changes of a peak area were recorded;
(7) the hydrolysis rates of Compound NX of the present application and the reference compound were observed and analyzed, and suitable compounds of the present application were screened out based on data.

3.2 Compound NX of the present application and the reference compound were metabolized in plasma for 120 min. Test results are shown in Table 3.

**Table 3**

| Compound | Remaining Amount of the Prodrug Compound (%) | Amount of Active Metabolites Produced (%) |
|---|---|---|
| N2 | 87.4±5.3 | 10.7±0.4 |
| N2S | 78.6±2.6 | 16.9±0.8 |
| N2R | 87.6±3.6 | 7.8±0.3 |
| N6 | 58.96±3.1 | 27.6±1.1 |
| N6S | 40.5±1.6 | 44.2±1.5 |
| N6R | 74.6±1.4 | 18.4±0.7 |
| N9 | 62.5±1.3 | 20.3±0.8 |
| N9S | 45.0±2.1 | 36.2±1.6 |
| N9R | 80.8±5.9 | 12.9±0.9 |
| N10 | 77.5±3.5 | 12.2±0.6 |
| N10S | 70.9±2.5 | 25.7±1.0 |
| N10R | 83.2±1.3 | 4.3±0.3 |
| FPA | 50.9±2.5 | 30.3±1.8 |
| S-FPA | 37.4±1.9 | 44.3±2.3 |

As can be seen from the test results, an S-isomer (abbreviated as NXS) of Compound NX has a faster metabolic rate than the racemate (abbreviated as NX), that is, a dexibuprofen ester prodrug has a faster hydrolysis rate in plasma than a racemic ibuprofen ester prodrug and can be converted into active metabolites in in vitro human plasma more quickly than a traditional racemic ibuprofen ester prodrug, so as to exert pharmacological activity.

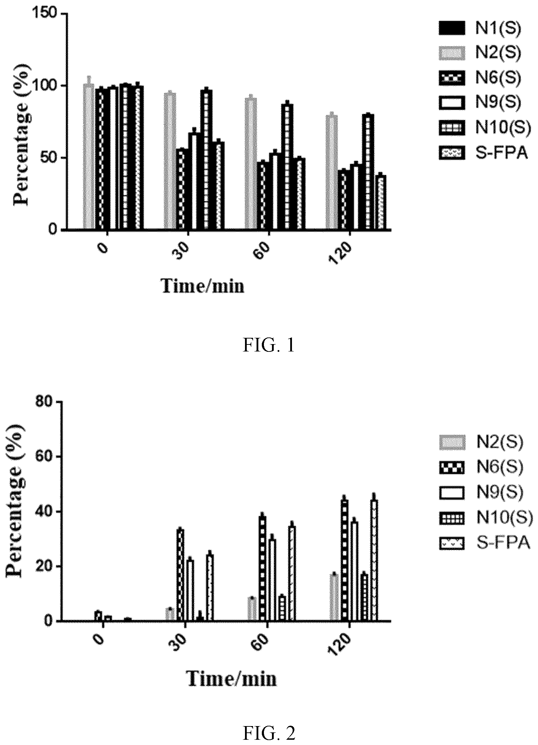

The degradation rates of the S-isomers of the compounds of the present application in human plasma at different time points (0, 15, 30, 60, and 120 min) are shown in FIG. 1, and the production rates of active metabolites are shown in FIG. 2. As can be seen from the figures, Compound N6S and N9S have relatively fast metabolic rates close to that of S-FPA; and Compound N2S and N10S have relatively slow metabolic rates since the compounds have relatively large steric hindrance and are difficult to combine with the corresponding esterase and hydrolyze, as speculated by the applicant.

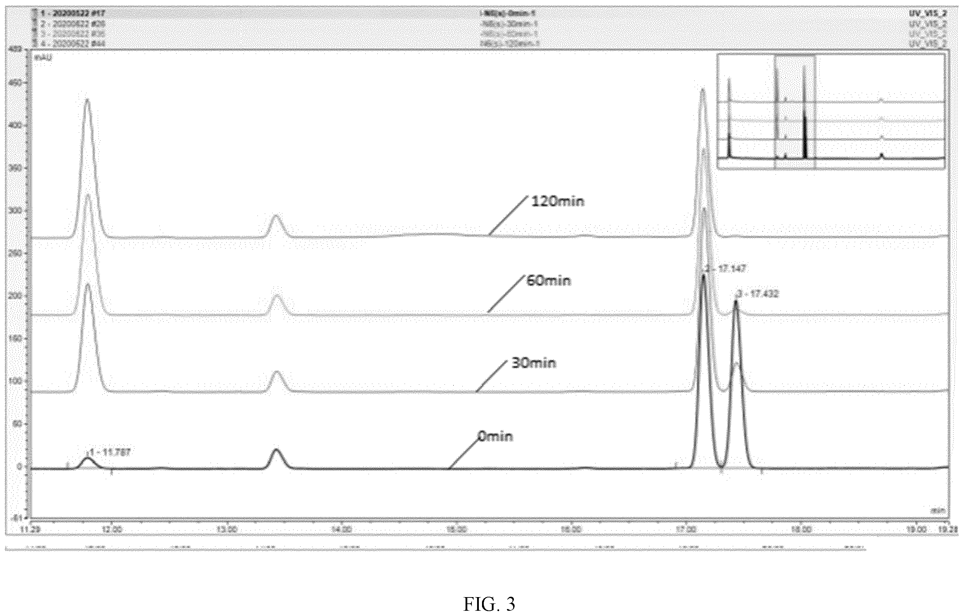

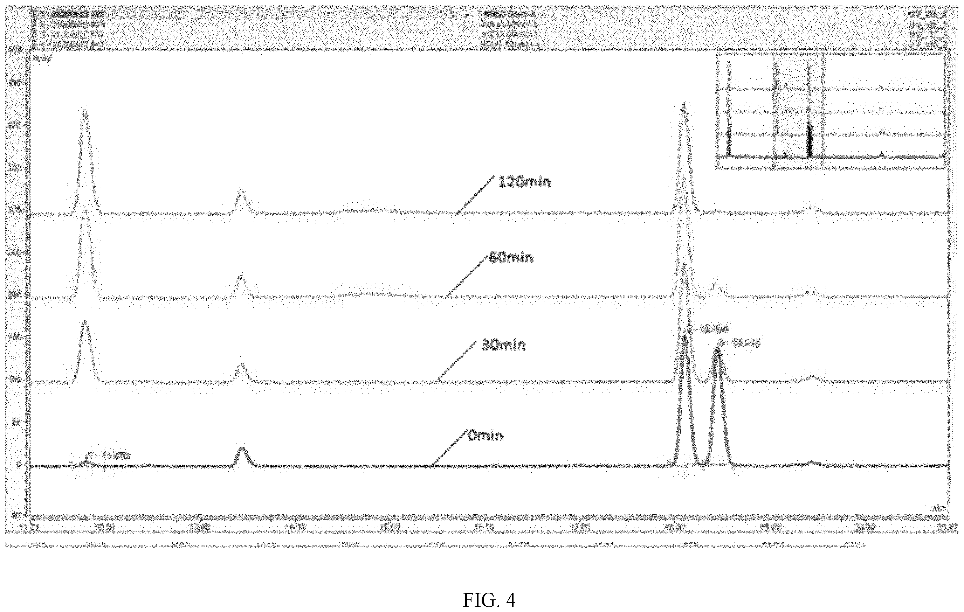

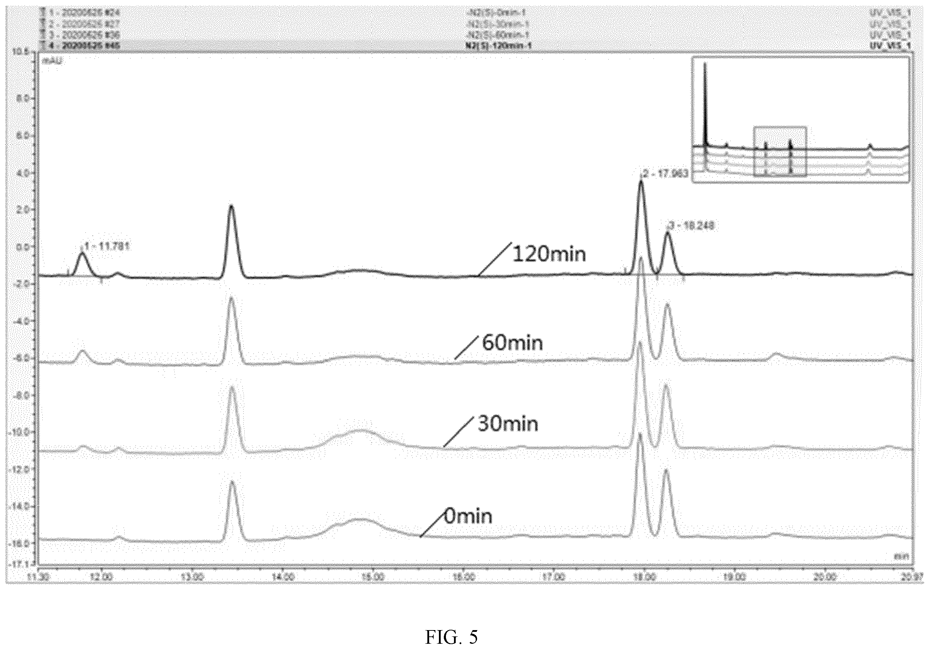

Meanwhile, the applicant has also discovered during the study that Compound N6(S) contains two peaks, indicating that N6(S) further contains a chiral center at a link to a side chain in addition to a chiral center on main chain ibuprofen, and the corresponding Compounds N6(S)-1 and N6(S)-2 have different degradation rates (FIG. 3). Similarly, two peaks contained in Compound N9(S), that is, the corresponding Compounds N9(S)-1 and N9(S)-2, have different degradation rates (FIG. 4). It indicates that two isomers in N6(S) or N9(S) are hydrolyzed at different rates by carboxylesterase in human plasma. For other compounds such as N2(S), two peaks have little difference in changing due to a relatively slow degradation rate (FIG. 5).

Compounds N6(S)-1 and N6(S)-2 may be obtained through preparation and separation of Compound N6(S) with a reversed-phase C18 chromatographic column, which have the following specific structural formulas:

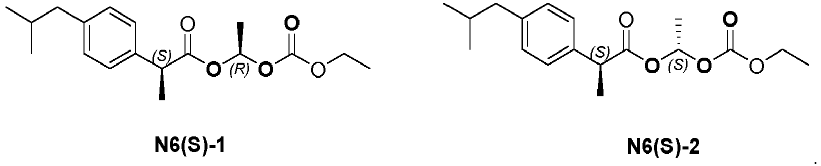

Compounds N9(S)-1 and N9(S)-2 are obtained through preparation and separation of Compound N9(S) with a reversed-phase C18 chromatographic column, which have the following specific structural formulas:

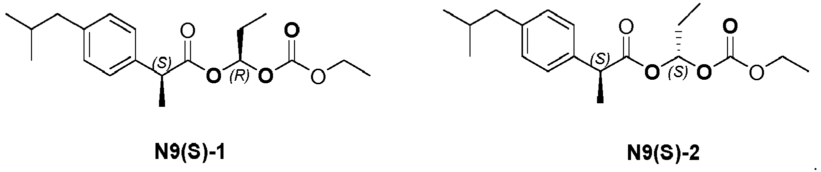

### Test Example 4: Study on pharmacokinetics of the compound of the present application in rats

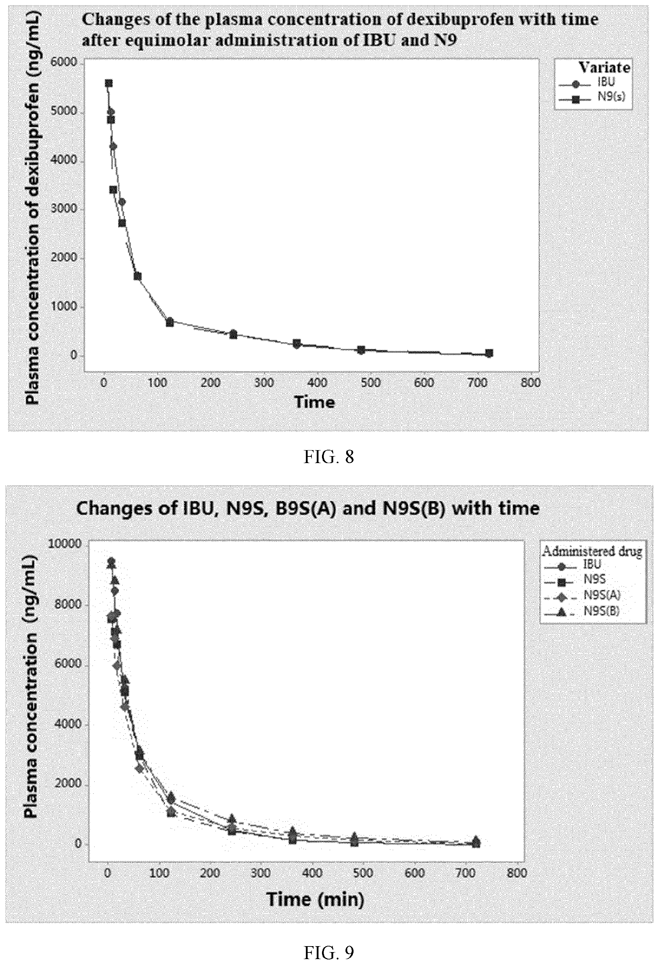

After the dexibuprofen ester derivative N9(S) (solubilized by PEG 400) of the present application and dexibuprofen (arginine solution) were administered to rats through tail vein injection at an equimolar dose, blood was collected from the eye fundus vein in 5, 10, 15, 30, 60, 120, 240, 360, 480, and 720 min after administration and put in a test tube treated with heparin. The whole blood was centrifuged at 8000 rpm for 5 min, and plasma samples were taken and stored at -80 °C, separately. The concentration of dexibuprofen (IBU) in plasma was analyzed and determined. Changes of dexibuprofen in rat plasma with time were investigated. FIG. 8 shows plasma concentration-time curves of injections of dexibuprofen and the dexibuprofen ester derivative N9(S). Through the analysis by pharmacokinetic software, the pharmacokinetic parameters were obtained, as shown in Table 5.

Preparation of test solutions: the test solutions were administered through tail veins of rats at a volume of 0.25 mL/100 g.

Dexibuprofen to be tested: an dexibuprofen arginine solution containing dexibuprofen of 20 mg/mL was prepared; 200 µL of the solution was pipetted into a 10 mL EP tube, added with 4800 µL of normal saline with a dispensing gun, and vortexed to be uniformly mixed so that a dosing solution was obtained, which had a concentration of 0.8 mg/mL and a volume of 5 mL.

N9 to be tested: the dexibuprofen ester derivative N9 (20 mg) was added into a 15 mL EP tube, added with 10 mL of PEG 400 with a dispensing gun, and vortexed to be uniformly mixed so that a PEG solution of the compound was obtained, which had a concentration of 2 mg/mL. The PEG solution (3250 µL) of N9 was pipetted into a 10 mL EP tube, added with 1750 µL of normal saline with the dispensing gun, and vortexed to be uniformly mixed so that a dosing solution was obtained, which had a concentration of 1.3 mg/mL and a volume of 5 mL.

**Table 4**

| Gro up | Test Substance | Molecular Weight | Dosage Ratio (Molecular Mass Ratio) | Dose (converted to rats) |
|---|---|---|---|---|
| | | g/mol | | mg/kg |
| 1 | Dexibuprofen | 206.28 | 1 | 2 |
| 2 | N9 | 336.43 | 1.63 | 3.26 |

**Table 5**

| Parameter | Dexibuprofen | N9S |
|---|---|---|
| T_{1/2} (min) | 114.68±15.54 | 145.98±18.48 |
| AUCo-t (min^{∗}ng/mL) | 422847.52±26422.43 | 385931.58±46873.23 |
| Cl (mL/min/kg) | 4.70±0.31 | 4.32±0.93 |
| MRT₀₋ₜ (min) | 122.61±19.93 | 157.31±16.67 |
| V_{SS} (mL/kg) | 572.40±65.75 | 1314.72±227.31 |

As can be seen from the test results, the drug-time curve for the dexibuprofen ester derivative prodrug N9 which is converted into dexibuprofen in the rat body basically overlaps with the drug-time curve for a dexibuprofen salt solution directly injected, and AUCo-t has no significant difference. It can be seen that the ester prodrug can be metabolized quickly into dexibuprofen with anti-inflammatory activity in rats, and the overall exposure in rats is consistent. The pharmacokinetic parameters show that the replacement of a dexibuprofen salt with an ester prodrug in injection preparations can prolong the half-life and mean residence time of dexibuprofen, reduce an elimination rate of dexibuprofen, and increase an apparent volume of distribution. Therefore, the present application prolongs the time of action of the drug in the body while achieving the same efficacy in clinical applications.

### Test Example 5

The dexibuprofen derivative Compound N9(S) includes a chiral center at the position where a side chain is joined in addition to a chiral center on main chain ibuprofen. Therefore, PEG 400 solubilized solutions of a side chain racemic compound of the dexibuprofen derivative Compound N9(S) and side chain chiral compounds N9(S)A and N9(S)B and dexibuprofen (aqueous arginine solution) were administered through tail veins at an equimolar dose, and the changes of dexibuprofen production in rat plasma with time were monitored, so as to investigate differences in metabolism pharmacokinetics (prodrug conversion) between two isomers which have isomeric side chains of ester and dextrorotatory main chains. Through the analysis by pharmacokinetic software, FIG. 9 shows plasma concentration-time curves of injections of dexibuprofen, the dexibuprofen ester derivative N9(S) and side chain chiral compound N9(S)A and N9(S)B.

Method for preparing test solutions: the dexibuprofen derivative Compound N9(S) (20 mg), a side chain isomer N9(S)A (20 mg) and a side chain isomer N9(S)B (20 mg) were each added into a 15 mL EP tube, added with 10 mL of PEG 400 with a dispensing gun, and vortexed to be uniformly mixed so that PEG solutions of the compounds were obtained, each of which had a concentration of 2 mg/mL. The PEG solution (3250 µL) of the compound to be tested was pipetted into a 10 mL EP tube, added with 1750 µL of normal saline with the dispensing gun, and vortexed to be uniformly mixed so that a dosing solution was obtained, which had a concentration of 1.3 mg/mL and a volume of 5 mL.

Method for preparing dexibuprofen: an dexibuprofen arginine solution containing dexibuprofen of 20 mg/mL was prepared; 200 µL of the solution was pipetted into a 10 mL EP tube, added with 4800 µL of normal saline with a dispensing gun, and vortexed to be uniformly mixed so that a dosing solution was obtained, which had a concentration of 0.8 mg/mL and a volume of 5 mL.

**Table 6**

| No. | Test compound | Dosing Solution | Dosage (mg/kg) | Volume of Administration through Tail Veins of Rats |
|---|---|---|---|---|
| Group 1 | N9(S) | PEG solution of N9(S) diluted with normal saline | 3.26 | 0.25 mL/100 g |
| Group 2 | N9(S)A | PEG solution of N9(S)A diluted with normal saline | 3.26 | 0.25 mL/100 g |
| Group 3 | N9(S)B | PEG solution of N9(S)B diluted with normal saline | 3.26 | 0.25 mL/100 g |
| Group 4 | Dexibuprofen | Dexibuprofen arginine solution diluted with normal saline | 2 | 0.25 mL/100 g |

Compounds N9(S)A and N9(S)B are obtained through preparation and separation of Compound N9(S) with a reversed-phase C18 chromatographic column, which have the following specific structural formulas:

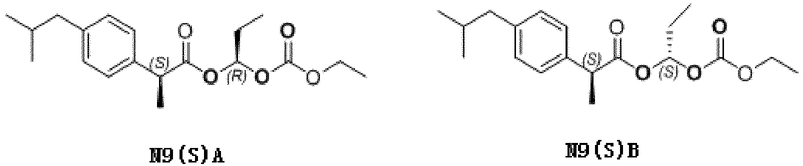

The results of the pharmacokinetic study show that three test compounds of dexibuprofen can all be rapidly metabolized at an equimolar dose.

### Test Example 6: Study on the stability of a dextrorotatory enantiomer of the compound in a solvent

4.1 Test scheme: Compounds N6(S) and N9(S) were dissolved in medium chain triglycerides (MCT), soybean oil, and an aqueous solution of a surfactant with a certain concentration (a phosphate buffer of Tween 80), separately. Samples were placed at 80 °C, and samples were taken on Day 0, 5, and 10 to determine the purity of the compounds and changes of the related substance (ibuprofen) and an isomer (levorotatory).

**Table 7 Sample formulations for investigation of solution stability**

| | Total Prepara tion Amount (g) | Concentrat ion of the Drug (mg/g) | Amount of the Drug (g) | Amount of a Single Detection Sample (g) | Amount of the Solvent (g) | Amount of Tween 80 (g) |
|---|---|---|---|---|---|---|
| Drug -containing MCT solution | 12 | 50 | 0.6 | 1 | 11.4 | / |
| Drug -containing soybean oil solution | 12 | 50 | 0.6 | 1 | 11.4 | / |
| Drug -containing surfactant aqueous solution | 80 | 5 | 0.4 | 6 | 59.6 | 20 |

4.2 Determination of the related substance in accordance with high-performance liquid chromatography (Chinese Pharmacopoeia 2015 Edition General Rules 0512)
(1) Chromatographic conditions: Chromatographic column: Agilent ZORBAX SB-C8, 4.6 mm×250 mm, 5 µm; mobile phase: 0.1% phosphoric acid solution-acetonitrile (50:50); detection wavelength: 220 nm; flowrate: 1.0 mL per minute; column temperature: 40 °C.
(2) Determination method: An appropriate amount of the substance was taken, accurately weighed, added with a diluent [anhydrous ethanol], shaken to be dissolved, quantitatively diluted into a solution of about 0.4 mg/mL, and vortexed for 30s. The solution was filtered with a PTFE filter membrane (0.22 µm, Jinteng) and the filtrate was used as the test solution. 10 µL was accurately measured into a liquid chromatograph and the chromatogram was recorded. In the chromatogram of the test solution, if there were impurity peaks, the results should be obtained by calculation according to a peak area normalization method.

4.3 Determination of the isomer in accordance with high-performance liquid chromatography (Chinese Pharmacopoeia 2015 Edition General Rules 0512)

Chromatographic conditions: filler: amylose-tris(3,5-dimethylphenylcarbamate) silica gel (Chiralpak AD-H, 250×4.6 mm, 5 µm); mobile phase: n-hexane-isopropanol (90:10) for isocratic elution of about 10 min; flowrate: 1.0 mL per minute; column temperature: 25 °C; detection wavelength: 219 nm.

Determination method: An appropriate amount of the isomer was taken, accurately weighed, added with a diluent [n-hexane-isopropanol (90:10)], shaken to be dissolved, quantitatively diluted into a solution of about 1.0 mg/mL, and vortexed for 30s. The solution was filtered with a PTFE filter membrane (0.22 µm, Jinteng) and the filtrate was used as the test solution. 10 µL was accurately measured into a liquid chromatograph and the chromatogram was recorded. In the chromatogram of the test solution, if there were isomer peaks, the results should be obtained by calculation according to a peak area normalization method.

The test results are shown in Table 5.

**Table 8**

| Compou nd | Name and Batch Number | Placing Condition | Time | Ibuprofen (%) | Purity of related Main Peak (%) | Isomer (%) |
|---|---|---|---|---|---|---|
| N6(S) | HR2001-N6(S) MCT | 80 °C | Day 0 | 0.03 | 99.89 | 3.26 |
| | | | Day 5 | 0.05 | 99.88 | 3.47 |
| | | | Day 10 | 0.06 | 99.87 | 3.45 |
| | HR2001-N6(S) Soybean oil | 80 °C | Day 0 | 0.03 | 99.84 | 3.26 |
| | | | Day 5 | 0.08 | 99.79 | 3.21 |
| | | | Day 10 | 0.16 | 99.47 | 3.20 |
| | HR2001-N6(S) Phosphate buffer of Tween 80 | 80 °C | Day 0 | 2.42 | 97.56 | 3.68 |
| | | | Day 5 | 10.24 | 89.66 | 3.97 |
| | | | Day 10 | 16.98 | 82.73 | 3.88 |
| N9(S) | HR2001-N9(S) MCT | 80 °C | Day 0 | / | 99.80 | 0.95 |
| | | | Day 5 | 0.02 | 99.78 | 0.95 |
| | | | Day 10 | 0.04 | 99.66 | 0.94 |
| | HR2001-N9(S) Soybean oil | 80 °C | Day 0 | / | 99.85 | 0.91 |
| | | | Day 5 | 0.09 | 99.57 | 0.90 |
| | | | Day 10 | 0.13 | 99.38 | 0.97 |
| | HR2001-N9(S) Phosphate buffer of Tween 80 | 80 °C | Day 0 | 0.63 | 99.03 | 0.86 |
| | | | Day 5 | 5.27 | 92.78 | 1.19 |
| | | | Day 10 | 8.76 | 87.97 | 1.78 |

As can be seen from the test results, after dexibuprofen ester compounds N6(S) and N9(S) of the compound of the present application are placed in an oil phase or water phase at a high temperature of 80 °C for 10 days, the purity of the isomer remains basically unchanged; and the related substances of the two compounds in the oil phase remain basically unchanged (relatively high stability), but the content of the related substance in the water phase increases.

Embodiments of the present application are described above. However, the present application is not limited to the preceding embodiments. Any modifications, equivalent substitutions, improvements, and the like made within the spirit and principle of the present application should fall within the scope of the present application.

## Claims

1. A compound represented by Structural Formula (1), a racemate, stereoisomer or pharmaceutically acceptable salt or solvate thereof, or a solvate of a pharmaceutically acceptable salt thereof; 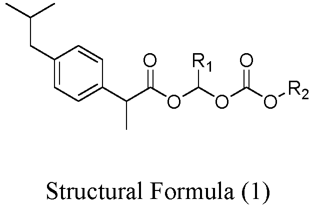 wherein, R₁ and R₂ are identical or different and are each independently selected from C₁₋₁₀ alkyl, C₂₋₁₀ alkenyl, C₂₋₁₀ alkynyl, C₃₋₈ cycloalkyl, 5-10 membered heterocyclic groups, C₆₋₁₀ aryl, and 5-10 membered heteroaryl groups; wherein C₁₋₁₀ alkyl, C₂₋₁₀ alkenyl, C₂₋₁₀ alkynyl, C₁₋₁₀ alkoxy, C₃₋₈ cycloalkyl, C₃₋₈ cycloalkyloxy, 5-10 membered heterocyclic groups, C₆₋₁₀ aryl, and 5-10 membered heteroaryl groups can be optionally substituted by one, two or more halogens, hydroxyl groups, amino groups, C₁₋₈ alkyl groups, C₂₋₈ alkenyl groups, C₂₋₈ alkynyl groups, C₁₋₈ alkoxy groups, C₆₋₁₀ aryl groups optionally substituted by C₁₋₁₀ alkyl, or 5-10 membered heteroaryl groups optionally substituted by C₁₋₁₀ alkyl.

2. The compound according to claim 1, wherein R₁ and R₂ are identical or different and are each independently selected from C₁₋₆ alkyl, C₂₋₆ alkenyl, C₂₋₆ alkynyl, C₃₋₈ cycloalkyl, 5-8 membered heterocyclic groups, C₆₋₈ aryl, and 5-8 membered heteroaryl groups; wherein C₁₋₆ alkyl, C₂₋₆ alkenyl, C₂₋₆ alkynyl, C₁₋₆ alkoxy, C₃₋₈ cycloalkyl, C₃₋₈ cycloalkyloxy, 5-8 membered heterocyclic groups, C₆₋₈ aryl, and 5-8 membered heteroaryl groups can be optionally substituted by one, two or more halogens, hydroxyl groups, amino groups, C₁₋₆ alkyl groups, C₂₋₆ alkenyl groups, C₂₋₆ alkynyl groups, C₁₋₆ alkoxy groups, C₆₋₈ aryl groups optionally substituted by C₁₋₆ alkyl, or 5-8 membered heteroaryl groups optionally substituted by C₁₋₆ alkyl.

3. The compound according to claim 1 or 2, wherein R₁ and R₂ are identical or different and are each independently selected from C₁₋₆ alkyl, C₂₋₆ alkenyl, C₂₋₆ alkynyl, and C₃₋₈ cycloalkyl, wherein C₁₋₆ alkyl, C₂₋₆ alkenyl, C₂₋₆ alkynyl, and C₃₋₈ cycloalkyl can be optionally substituted by one, two or more fluorine, chlorine, bromine, iodine, hydroxyl groups, amino groups, or phenyl groups optionally substituted by C₁₋₆ alkyl.

4. The compound according to any one of claims 1 to 3, wherein R₁ is methyl, ethyl, propyl, isopropyl, n-butyl, isobutyl, tert-butyl, cyclopropyl, cyclobutyl, cyclopentyl, or cyclohexyl; andR₂ is methyl, ethyl, propyl, isopropyl, n-butyl, tert-butyl, isobutyl, cyclopropyl, cyclobutyl, cyclopentyl, or cyclohexyl.

5. The compound according to any one of claims 1 to 4, wherein the compound represented by Structural Formula (1) is selected from the following compounds and dextrorotatory enantiomers thereof, that is, isomers whose carbon at position 1 is in an S configuration:

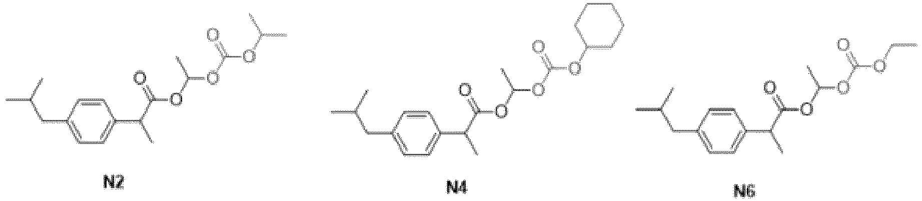

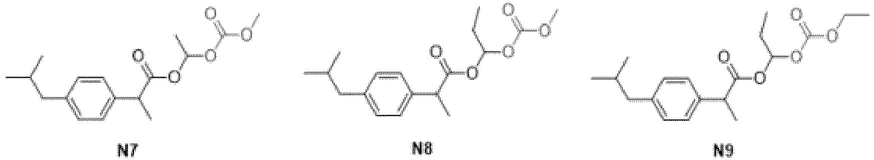

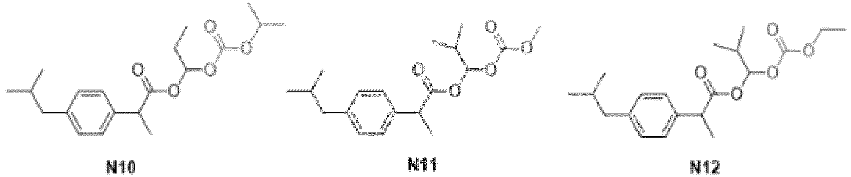

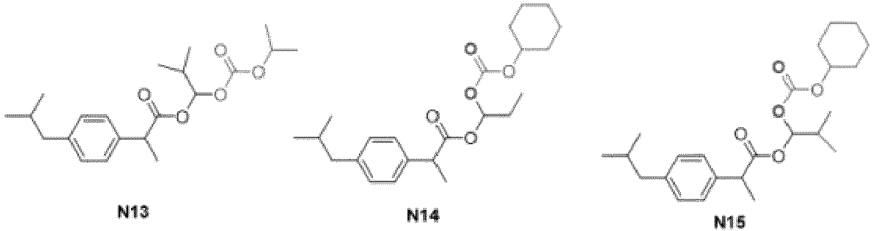

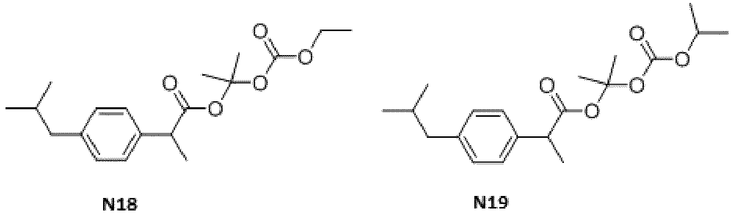

6. The compound according to any one of claims 1 to 5, the racemate, stereoisomer or pharmaceutically acceptable salt or solvate thereof, or the solvate of a pharmaceutically acceptable salt thereof, wherein the compound represented by Structural Formula (I) is selected from Compounds N2, N6, N9, N10, N18, N19 and dextrorotatory enantiomers thereof, that is, isomers whose carbon at position 1 is in an S configuration, wherein the dextrorotatory enantiomers have the following structures:

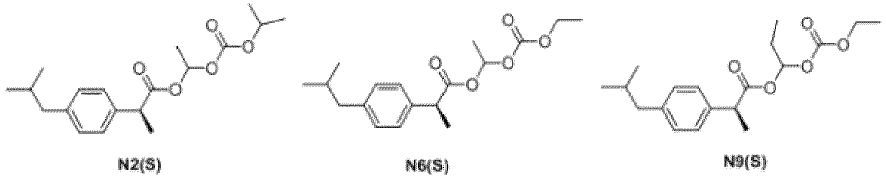

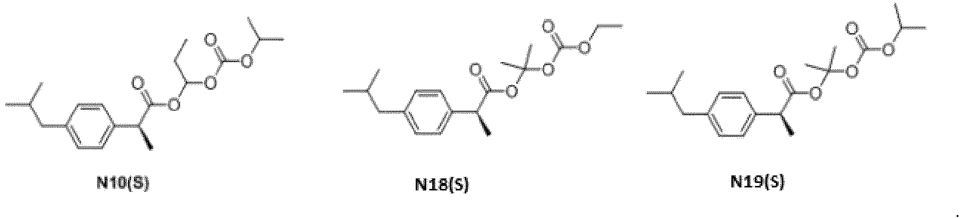

7. The compound according to any one of claims 1 to 5, the racemate, stereoisomer or pharmaceutically acceptable salt or solvate thereof, or the solvate of a pharmaceutically acceptable salt thereof, wherein the compound represented by Structural Formula (I) is selected from Compounds N4, N14 and dextrorotatory enantiomers thereof, that is, isomers whose carbon at position 1 is in an S configuration, wherein the dextrorotatory enantiomers have the following structures:

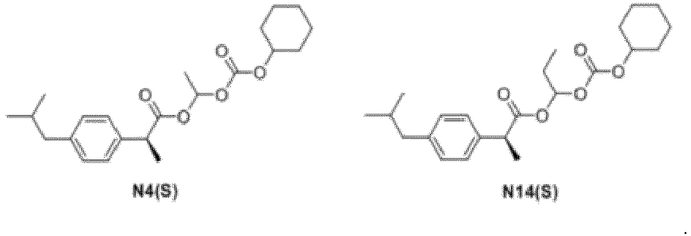

8. The compound according to claim 6, the racemate, stereoisomer or pharmaceutically acceptable salt or solvate thereof, or the solvate of a pharmaceutically acceptable salt thereof, wherein the compound represented by Structural Formula (I) is selected from Compounds N6(S) and N9(S);
optionally, Compound N6(S) is a mixture of the following compounds N6(S)-1 and N6(S)-2 which are mixed at any ratio: 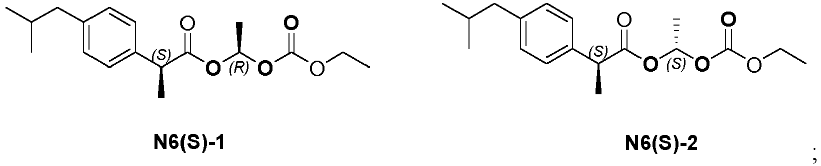
optionally, Compound N9(S) is a mixture of the following compounds N9(S)-1 and N9(S)-2 which are mixed at any ratio:

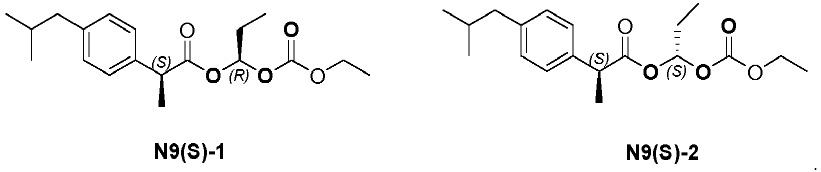

9. A method for preparing the compound according to any one of claims 1 to 8, comprising a step of reacting Compound 1 with an organic carbonate represented by Structural Formula (2): 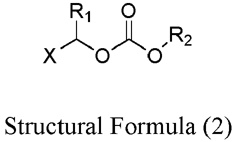
wherein in Structural Formula (2), X is chlorine, bromine, or iodine, and R₁ and R₂ have meanings defined in any one of claims 1 to 3;
wherein Compound 1 is racemic ibuprofen or ibuprofen with an S or R configuration, that is, (±)-2-(4-isobutylphenyl)propionic acid, (S)-2-(4-isobutylphenyl)propionic acid, or (R)-2-(4-isobutylphenyl)propionic acid;
optionally, the reaction is conducted in the presence of an acid-binding agent.

10. The method according to claim 9, comprising the following steps: putting the ibuprofen and the acid-binding agent in a reaction vessel, adding a reaction solvent to mix, then adding a compound represented by Structural Formula (2) 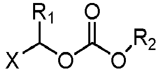 to the reaction vessel, and stirring the reaction solution after the addition; then, extracting the reaction solution and washing, drying an organic phase and concentrating, and purifying through column chromatography to obtain a target compound.

11. The method according to claim 9 or 10, wherein the acid-binding agent may be an inorganic base such as NaOH, KOH, K₂CO₃, KHCO₃, Na₂CO₃, and NaHCO₃ or an organic base such as one, two or more of triethylamine, pyridine, DMAP, DIEA, or DBU; the reaction may be conducted at a temperature of -5-80 °C for a time of 0.5-24 h; and the reaction solvent may be one, two or more of acetone, dichloromethane, trichloromethane, carbon tetrachloride, tetrahydrofuran, acetonitrile, DMF, DMAc, or ether.

12. The method according to any one of claims 9 to 11, wherein a halogenated organic carbonate represented by Structural Formula (2) is prepared by the following method: firstly reacting an organic aldehyde R₁CHO with triphosgene at a low temperature to obtain a chloroalkyl formate intermediate, and then reacting the chloroalkyl formate with an organic alcohol R₂-OH to obtain a chlorinated organic carbonate; and optionally, further reacting the chlorinated organic carbonate with NaI to obtain an iodo organic carbonate; wherein the reaction formula is: 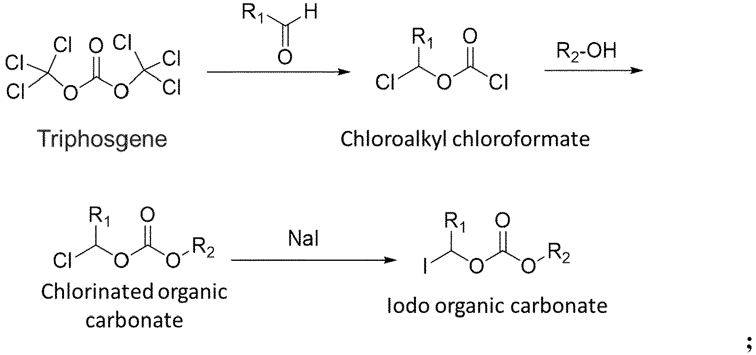 wherein, R₁ and R₂ each have meanings defined in any one of claims 1 to 4.

13. Use of the compound represented by Structural Formula (1) according to any one of claims 1 to 8, the racemate, stereoisomer or pharmaceutically acceptable salt or solvate thereof, or the solvate of a pharmaceutically acceptable salt thereof in preparation of a drug; optionally, the drug is used for treatment of one or more of the following diseases: rheumatoid arthritis, low back pain, migraine, neuralgia, periarthritis of shoulder or osteoarthritis, anti-inflammation and/or analgesia of neck-shoulder-wrist syndromes, analgesia and/or anti-inflammation after surgery, trauma or tooth extraction, and antipyretic and/or analgesia of acute upper respiratory tract inflammation.

14. A pharmaceutical composition, comprising the compound represented by Structural Formula (1) according to any one of claims 1 to 8, the racemate, stereoisomer or pharmaceutically acceptable salt or solvate thereof, or the solvate of a pharmaceutically acceptable salt thereof; optionally, the pharmaceutical composition is an oral preparation such as tablets, capsules, and granules, injections, eye drops, gels, creams, ointments, or cataplasm, for example, a fat emulsion injection.
